(19) Europäisches Patentamt
European Patent Office
Office européen des brevets

(11) **EP 4 442 363 A1**

(12) **EUROPEAN PATENT APPLICATION**
published in accordance with Art. 153(4) EPC

(43) Date of publication:
**09.10.2024 Bulletin 2024/41**

(21) Application number: **22901288.5**

(22) Date of filing: **29.11.2022**

(51) International Patent Classification (IPC):
**B01J 23/887** (2006.01)    **B01J 35/10** (2006.01)
**C07B 61/00** (2006.01)    **C07C 45/35** (2006.01)
**C07C 47/22** (2006.01)

(52) Cooperative Patent Classification (CPC):
**B01J 23/887; B01J 35/60; C07B 61/00;
C07C 45/35; C07C 47/22**

(86) International application number:
**PCT/JP2022/043923**

(87) International publication number:
**WO 2023/100856 (08.06.2023 Gazette 2023/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.11.2021 JP 2021193719**

(71) Applicant: **Nippon Kayaku Kabushiki Kaisha
Tokyo 100-0005 (JP)**

(72) Inventors:
• **OKUMURA, Shigeki
Sanyoonoda-shi, Yamaguchi 757-8686 (JP)**
• **KAGAWA, Tsukasa
Sanyoonoda-shi, Yamaguchi 757-8686 (JP)**
• **YASUDA, Shogo
Sanyoonoda-shi, Yamaguchi 757-8686 (JP)**

(74) Representative: **Gille Hrabal
Partnerschaftsgesellschaft mbB
Patentanwälte
Brucknerstraße 20
40593 Düsseldorf (DE)**

(54) **CATALYST, AND METHOD FOR PRODUCING COMPOUND USING SAME**

(57)    The present invention relates to a catalyst having at least one peak in each of a range of a pore diameter of 0.45 μm to 10.0 μm and a range of a pore diameter of 0.040 μm to 0.45 μm in a pore distribution (-dV/d (log D) vs D) measured by mercury porosimetry, in which when a maximum value of -dV/d (log D) in the range of the pore diameter of 0.45 μm to 10.0 μm is represented by A and a maximum value of -dV/d (log D) in the range of the pore diameter of 0.040 μm to 0.45 μm is represented by B, (B/A) is 0.20 to 13.0 (V represents a volume (unit: mL/g) of filled mercury per sample weight, D represents the pore diameter (unit: μm), log D represents a natural logarithm of D, and -dV/d (log D) represents a reciprocal of a value obtained by differentiating a cumulative pore volume in a specific pore diameter range by a logarithm of the pore diameter).

**FIG. 1**

EP 4 442 363 A1

**Description**

TECHNICAL FIELD

The present invention relates to a novel catalyst having high activity and achieving obtaining a target product with high yield, and more particularly, to a catalyst capable of stably oxidatively producing, with high yield, an unsaturated aldehyde, an unsaturated carboxylic acid, or a conjugated diene even in a region having high catalytic activity.

BACKGROUND ART

A method for producing a corresponding unsaturated aldehyde or unsaturated carboxylic acid using propylene, isobutylene, t-butyl alcohol, or the like as a raw material, and a gas-phase catalytic oxidation method for producing 1,3-butadiene from butenes are widely practiced industrially, and use of a composite metal oxide catalyst containing bismuth and molybdenum as main components in these methods is known to those skilled in the art.

In particular, regarding the method for producing a corresponding unsaturated aldehyde or unsaturated carboxylic acid using propylene, isobutylene, t-butyl alcohol, or the like as a raw material, many reports have been made as means for improving the yield (for example, Patent Literatures 1 and 2). Even when improvement is made by the means as described above, further improvement in yield is required in the production of a corresponding unsaturated aldehyde and/or unsaturated carboxylic acid by a partial oxidation reaction of propylene, isobutylene, t-butyl alcohol, or the like. For example, the yield of a target product influences an amount of propylene, isobutylene, t-butyl alcohol, or the like required for production and greatly influences the production cost.

To improve the productivity of the catalyst and reduce the cost of the catalyst, it is known to those skilled in the art to use a spray dryer at the time of drying a mother liquid obtained by mixing various catalyst raw materials in a blending step. For obtaining the composite metal oxide catalyst, for example, a mother liquid may be evaporated and dried or gelled as a method other than drying by a spray dryer. However, a semi-finished product after the step is thick or in a solidified state, and this makes handling the product difficult in a production method in an industrial scale and the cost of the catalyst tends to increase.

As disclosed in Patent Literature 3, a catalyst typically available for the composite metal oxide has a specific surface area of 5 $m^2$/g or more. Less than 5 $m^2$/g of the specific surface area causes decrease in a reaction rate, as a contact area of the catalyst and a raw material becomes small when the specific surface area is small, and as a result, the yield of the target product decreases. One of the methods of increasing the specific surface area is to increase a ratio of a pore volume having a small pore diameter to a total pore volume. For example, in Patent Literature 4, the yield is improved by increasing a ratio of a pore volume of a relatively small pore to a total pore volume. However, when the specific surface area is increased by reducing the pore diameter, the porosity inside the catalyst increases, and as a result, the strength of the catalyst may deteriorate. It is known to those skilled in the art that the catalyst having weak strength may not be usable in practical use as, for example, a part of the catalyst may lost at the time of filling the catalyst into a reaction tube, and that the catalyst having weak strength requires careful working at the time of filling to decrease the working efficiency.

Therefore, a strength aid is typically used in order to improve the strength, but a catalyst having high strength even without a strength aid has been desired due to an increase in cost of the strength aid itself and complexity of a step for charging the strength aid. As a method of increasing the strength without using a strength aid, for example, Patent Literature 5 describes a method for producing a catalyst having high strength by operating a relative centrifugal acceleration at the time of granulation in a tumbling granulation. However, this method achieves a high strength but cannot achieve a desired level of yield. That is, an advanced catalyst maintaining a required strength at the time of filling, containing no strength aid, and achieving a high yield at low cost cannot be implemented.

A cumulative pore volume (see Patent Literature 4) and a relation between catalyst performance and a ratio of the cumulative pore volume within a specific pore diameter range (see Patent Literature 3) are known. The mercury porosimetry measurement provides not only the cumulative pore volume but also information on a pore distribution with -dV/d (log D) as a vertical axis and D as a horizontal axis. V represents a volume (unit: mL/g) of the filled mercury per sample weight, D represents a pore diameter (unit: $\mu$m), log D represents a natural logarithm of D, and d represents a differential symbol. -dV/d (log D) in the pore distribution means a pore volume of a pore having a pore diameter of D, and when the distribution shape is steep in the pore distribution, it is indicated that there are many pores having a uniform pore diameter of D as compared with a case where the distribution shape is broad. Similarly, when -dV/d (log D) as the vertical axis is high in the pore distribution, it is indicated that there are many pores having a uniform pore diameter D as compared with a case where -dV/d (log D) is low. It has not been found so far how information related to a shape and a range of such a pore distribution relates to the catalyst performance and what numerical range of the above information the information indicates improves the catalyst performance.

CITATION LIST

PATENT LITERATURE

Patent Literature 1: WO2016/136882
Patent Literature 2: JP2017-024009A
Patent Literature 3: JPH06-007924B
Patent Literature 4: Japanese Patent No. 6447327
Patent Literature 5: Japanese Patent No. 5970542

SUMMARY OF INVENTION

TECHNICAL PROBLEM

The present invention proposes a catalyst having high selectivity and allowing for a high yield of a target product.

SOLUTION TO PROBLEM

According to the study of the present inventors, a composition and a production method of a catalyst have been investigated, focusing particularly on a distribution (-dV/d (log D) vs D) of a pore volume having a pore diameter of 0.45 $\mu$m to 10.0 $\mu$m and a distribution of a pore volume having a pore diameter of 0.040 $\mu$m to 0.45 $\mu$m, it has been found that a high yield can be achieved when (B/A) is in a specific range in the case where a maximum value of -dV/d (log D) in the range of the pore diameter of 0.45 $\mu$m to 10.0 $\mu$m is represented by A and a maximum value of -dV/d (log D) in the range of the pore diameter of 0.040 $\mu$m to 0.45 $\mu$m is represented by B, and the present invention has been completed. Accordingly, a sufficiently usable catalyst having a small specific surface area of less than 5 m$^2$/g has been completed.
That is, the present invention relates to the following 1) to 9).

1) A catalyst having at least one peak in a range of a pore diameter of 0.45 $\mu$m to 10.0 $\mu$m and at least one peak in a range of a pore diameter of 0.040 $\mu$m to 0.45 $\mu$m in a pore distribution (-dV/d (log D) vs D) measured by mercury porosimetry, in which
when a maximum value of -dV/d (log D) in the range of the pore diameter of 0.45 $\mu$m to 10.0 $\mu$m is represented by A and a maximum value of -dV/d (log D) in the range of the pore diameter of 0.040 $\mu$m to 0.45 $\mu$m is represented by B, (B/A) is 0.20 to 13.0, where V represents a volume (unit: mL/g) of filled mercury per sample weight, D represents the pore diameter (unit: $\mu$m), log D represents a natural logarithm of D, and -dV/d (log D) represents a reciprocal of a value obtained by differentiating a cumulative pore volume in a specific pore diameter range by a logarithm of the pore diameter.
2) The catalyst according to 1), in which a cumulative specific surface area is less than 5 m$^2$/g.
3)
The catalyst according to 1) or 2), containing molybdenum, bismuth, and iron.
4) The catalyst according to 3), having a catalyst composition represented by the following formula (1):

$$Mo_{a1}Bi_{b1}Ni_{c1}Co_{d1}Fe_{e1}X_{f1}Y_{g1}Z_{h1}O_{i1} \qquad (1),$$

where, Mo, Bi, Ni, Co, and Fe respectively represent molybdenum, bismuth, nickel, cobalt, and iron; X is at least one element selected from tungsten, antimony, tin, zinc, chromium, manganese, magnesium, silicon, aluminum, cerium, and titanium; Y is at least one element selected from sodium, potassium, cesium, rubidium, and thallium; Z belongs to Groups 1 to 16 in the periodic table, and means at least one element selected from elements other than the above Mo, Bi, Ni, Co, Fe, X, and Y; a1, b1, c1, d1, e1, f1, g1, h1, and i1 respectively represent the number of atoms of molybdenum, bismuth, nickel, cobalt, iron, X, Y, Z, and oxygen; and when a1 = 12, $0 < b1 \leq 7$, $0 \leq c1 \leq 10$, $0 < d1 \leq 10$, $0 < c1+d1 \leq 20$, $0 < e1 \leq 5$, $0 \leq f1 \leq 2$, $0 \leq g1 \leq 3$, $0 \leq h1 \leq 5$, and i1 is a value determined by an oxidation state of each element.
5) The catalyst according to any one of 1) to 4), in which the catalyst is carried on an inert carrier.
6) The catalyst according to 5), in which the inert carrier is silica and/or alumina.
7) The catalyst according to any one of 1) to 6), in which the catalyst is used for producing an unsaturated aldehyde compound, an unsaturated carboxylic acid compound, or a conjugated diene compound.
8) A method for producing an unsaturated aldehyde compound, an unsaturated carboxylic acid compound, or a conjugated diene compound including using the catalyst according to any one of 1) to 7).

9) The production method according to 8), in which the unsaturated aldehyde compound is acrolein, the unsaturated carboxylic acid compound is acrylic acid, and the conjugated diene compound is butadiene.

ADVANTAGEOUS EFFECTS OF INVENTION

The catalyst according to the present invention is highly effective in improving the raw material conversion rate and improving the yield in addition to maintaining sufficient mechanical strength in a gas-phase catalytic oxidation reaction.

BRIEF DESCRIPTION OF DRAWINGS

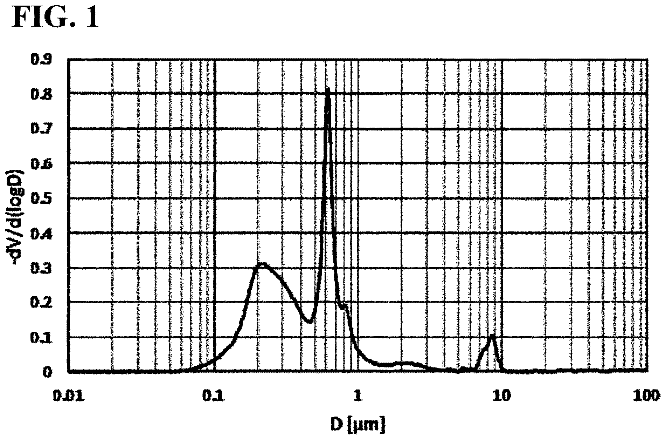

FIG. 1 is a chart illustrating a pore distribution of the present invention (Example 2).

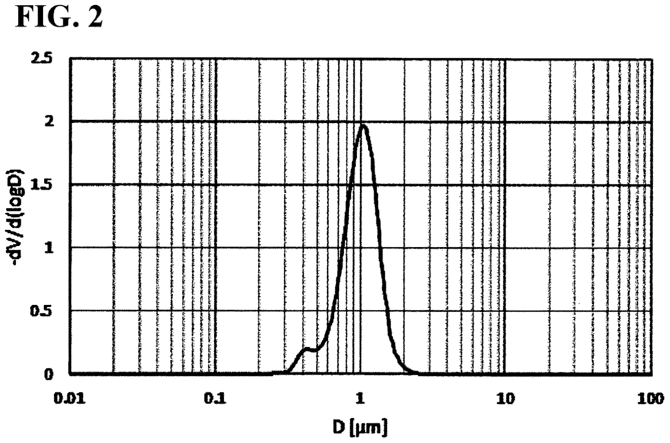

FIG. 2 is a chart illustrating a pore distribution of Comparative Example 1.

DESCRIPTION OF EMBODIMENTS

A catalyst according to the present invention is characterized in that, in a pore volume distribution (-dV/d (log D) vs D) measured by mercury porosimetry, when a maximum value of -dV/d (log D) in a range of a pore diameter of 0.45 $\mu$m to 10.0 $\mu$m is represented by A and a maximum value of -dV/d (log D) in a range of a pore diameter of 0.040 $\mu$m to 0.45 $\mu$m is represented by B, (B/A) is 0.20 to 13.0. V represents a volume (unit: mL/g) of filled mercury per sample weight, D represents the pore diameter (unit: $\mu$m), log D represents a natural logarithm of D, and d represents a differential symbol.

[Method for Measuring Pore Diameter and Pore Volume]

Results of the pore diameter and the pore volume in the present invention are obtained by measurement by mercury porosimetry using a porosimeter. Here, the mercury porosimetry is a method of applying a pressure to mercury, which has a high surface tension, filling mercury into pores and/or voids on a solid surface, determining a pore distribution based on a relation between the pressure applied at this time and a volume of the mercury filled into the pores and/or voids, and determining the pore diameter and the pore volume based on the pore distribution. One of the specific measurements is a method in which a catalyst is not subjected to pretreatment such as vacuum deaeration, a sample of the catalyst having a weight of about 5 g is put into a large cell (10 mm$\varphi$ $\times$ 6 cm) having a cell volume of 2 cc by using a fully automatic pore distribution measurement device (mercury porosimetry pore diameter analyzer Pore Master 60-GT manufactured by Anton Paar), mercury is filled into the cell, and a mercury pressure and a volume of the mercury filled into the pore are measured. The measurement is performed under conditions that a mercury surface tension is set to 480 dyn/cm, a mercury contact angle is set to 140°, a measurement temperature is set to 20°C, and a range of the pore diameter to be measured is set to 0.0036 $\mu$m to 400 $\mu$m. By assuming that all of the pores are cylindrical and performing analysis based on the pressure applied at the time of measurement and the Washburn's equation, the pore distribution of each pore diameter of the catalyst can be obtained.

[Method for Measuring Cumulative Specific Surface Area]

In the present invention, a cumulative specific surface area S is calculated by using the measurement result of the mercury porosimetry described above, converting the measurement result into a specific surface area distribution (-dS/d (log D) vs D) on the assumption that all the measured pores have a cylindrical shape, and then integrating the specific surface area distribution over all pore diameter ranges. Specifically, -dS/d (log D) is calculated by the following calculation formula from numerical data of -dV/d (log D) in each pore diameter D of the pore distribution (-dV/d (log D) vs D). In the following formula, Di is described for clearly indicating a calculation formula for a specific pore diameter, and a suffix of i means a number of a measurement point in the mercury porosimetry, and starts from 1 to the number of measurement points.

-   dS/d (log D)$_{D = Di}$ (unit: m$^2$/g) = -dV/d (log D)$_{D = Di}$ (unit: mL/g) $\div$ 1,000,000 (unit: mL/m$^3$) $\times$ 4 $\div$ (Di (unit: $\mu$m) $\div$ 1,000,000 (unit: $\mu$m/m))

The thus obtained -dS/d (log D)D = Di is calculated by integration over all measured pore diameter ranges, specifically by quadrature by parts, to obtain the cumulative specific surface area S (unit: m$^2$/g). A calculation formula of quadrature by parts is as follows.

[Formula 1]

$$S = \sum \left[ -\frac{dS}{d(logD)_{D=D_i}} \times \{(logD)_{D=D_{i-1}} - (logD)_{D=D_{i+1}}\} \div 2 \right]$$

Here, $\Sigma[\ ]$ is an operation that takes a sum of specific surface areas of all pore diameter ranges measured for D, excluding the cases of i = 1 and the maximum value (that is, the number of measurement points in the mercury porosimetry as described above). A reason for the exclusion of the cases of i = 1 and the maximum value is that there is no adjacent data at i = 1 and the maximum value (for example, there is no data (log D)D = D0 when i = 1).

[-dV/d (log D)]

The catalyst according to the present invention has a specific ratio regarding -dV/d (log D) (unit: mL/g) in a specific pore diameter range. V represents the volume (unit: mL/g) of the filled mercury per sample weight and D represents the pore diameter (unit: $\mu$m), and thus, -dV/d (log D) is a reciprocal of a value obtained by differentiating a cumulative pore volume in a specific pore diameter range by a logarithm of the pore diameter. That is, -dV/d (log D) can be considered as an index representing the pore volume distribution in a specific pore diameter D.

The present invention focuses on the range of the pore diameter of 0.45 $\mu$m to 10.0 $\mu$m and the range of the pore diameter of 0.040 $\mu$m to 0.45 $\mu$m as the specific range. The maximum value of -dV/d (log D) in the range of the pore diameter of 0.45 $\mu$m to 10.0 $\mu$m is represented by A, and the maximum value of -dV/d (log D) in the range of the pore diameter of 0.040 $\mu$m to 0.45 $\mu$m is represented by B.

[(B/A)]

In the catalyst according to the present invention, at least one peak is present in the pore diameter range of 0.040 $\mu$m to 0.45 $\mu$m and at least one peak is present in the pore diameter range of 0.45 $\mu$m to 10.0 $\mu$m in the pore distribution (-dV/d (log D) vs D), and (B/A) is 0.20 to 13.0.

An advantage of focusing on the pore diameter range is that when pores derived from primary particles in a pore diameter range of 0.040 $\mu$m to 0.45 $\mu$m and pores derived from secondary particles in a pore diameter range of 0.45 $\mu$m to 10.0 $\mu$m each represent a sharp pore distribution shape, the catalyst performance can be improved by controlling a ratio of the maximum values in the respective pore diameter regions to be within a certain range. In other words, the advantage is that the pore distribution derived from the primary particles and the pore distribution derived from the secondary particles have the similar distribution shape, or the number of pores derived from the primary particles and the number of the pores derived from the secondary particles are close or the same. The development has not been made in view of the above. That is, in the related art, the reaction activity as a catalyst has been improved by increasing the number of pores having a pore diameter smaller than 0.45 $\mu$m, but the mechanical strength of the catalyst has been also reduced at the same time as described above. However, the present inventors have found out that appropriately controlling a blending step and a drying step to be described later allows for controlling a pore distribution in the range of 0.45 $\mu$m to 10.0 $\mu$m, achieves a catalyst having high mechanical strength in addition to sufficient activity as a catalyst, and further surprisingly have found that the catalyst has high selectivity. Accordingly, the present inventors have completed the present invention. Here, the shape and the pore diameter of the pores derived from the primary particles are derived from dispersed particles formed in a blended solution in the blending step to be described later, and the shape and the pore diameter of the pores derived from the secondary particles are derived from a catalyst precursor formed in the drying step to be described later. That is, in the present invention, performing control to be described later in the blending step and the drying step to adjust the pore distribution of the primary particles and the pore distribution of the secondary particles, respectively, allows for exhibiting the desired catalytic performance. A numerical range of (B/A) of 0.20 to 13.0 is obtained by devising the composition and the production method, and thus the numerical range is not satisfied by a known catalyst. The specific pore distribution shape is achieved for the first time by the control of the blending step and the drying step to be described later, and this results in the catalyst according to the present invention. Therefore, the reason why the numerical range is not satisfied by a known catalyst is that the catalyst according to the present invention indicating the specific pore distribution shape is considered unusable in the related art.

An upper limit of (B/A) is 10.0, 8.0, 6.0, 4.0, 2.0, 1.8, 1.7, 1.6, 1.5, 1.2, and 1.0 in order of preference, and particularly preferably 0.9. A lower limit thereof is 0.3, 0.4, and 0.5 in order of preference, and particularly preferably 0.6. Therefore, a range of (B/A) is 0.2 to 10.0, 0.2 to 8.0, 0.2 to 6.0, 0.2 to 4.0, 0.2 to 2.0, 0.2 to 1.8, 0.2 to 1.7, 0.2 to 1.6, 0.3 to 1.5, 0.4 to 1.2, and 0.5 to 1.0 in order of preference, and a most preferred range is 0.6 to 0.9.

A preferred range of the maximum value (A) of -dV/d (log D) in the range of the pore diameter of 0.45 $\mu$m to 4.0 $\mu$m is 0.01 to 1.90, and a further preferred upper limit thereof is 1.75, 1.50, 1.25, and 1.00 in order, and particularly preferably 0.90. A lower limit thereof is 0.02, 0.05, 0.10, 0.15, 0.20, 0.25, and 0.30 in order of further preference, and particularly preferably 0.35. Therefore, a more preferred range is 0.02 to 1.90, 0.05 to 1.90, 0.10 to 1.90, 0.15 to 1.75, 0.20 to 1.50, 0.25 to 1.25, and 0.30 to 1.00 in order, and a most preferred range is 0.35 to 0.90.

A preferred range of the maximum value (B) of -dV/d (log D) in the range of 0.040 $\mu$m to 0.45 $\mu$m is 0.01 to 1.00, and a further preferred upper limit thereof is 0.90, 0.80, 0.70, and 0.60 in order, and particularly preferably 0.50. A lower limit thereof is 0.05, 0.10, 0.15, 0.18, 0.19, and 0.20 in order of further preference, and particularly preferably 0.23. Therefore, a more preferred range is 0.05 to 1.00, 0.10 to 1.00, 0.15 to 0.90, 0.18 to 0.80, 0.19 to 0.70, and 0.20 to 0.60 in order, and a most preferred range is 0.23 to 0.50.

[(D/C)]

Preferably, the catalyst according to the present invention has at least one peak in a pore diameter range of 0.040 $\mu$m to 0.30 $\mu$m and has at least one peak in a pore diameter range of 0.30 $\mu$m to 10.0 $\mu$m in a differential pore volume distribution (dV/dD vs D), and when a maximum value of dV/dD in the pore diameter range of 0.30 $\mu$m to 10.0 $\mu$m is represented by C and a maximum value of dV/dD in the pore diameter range of 0.040 $\mu$m to 0.30 $\mu$m is represented by D, (D/C) is 0.01 to 1,000.00. V represents the volume (unit: mL/g) of the filled mercury per sample weight, D represents the pore diameter (unit: $\mu$m), and d represents the differential symbol. The unit of dV/dD is mL/(g·$\mu$m).

An upper limit of (D/C) is 500.00, 100.00, 75.00, 50.00, 25.00, 10.00, 5.00, and 4.50 in order of preference, and particularly preferably 4.20. A lower limit thereof is 0.05, 0.10, 0.20, and 0.30 in order of preference, and particularly preferably 0.40. Therefore, a range thereof is 0.01 to 500.00, 0.01 to 100.00, 0.01 to 75.00, 0.01 to 50.00, 0.05 to 25.00, 0.10 to 10.00, 0.20 to 5.00, and 0.30 to 4.50 in order of more preference, and a most preferred range thereof is 0.40 to 4.20.

A preferred range of a maximum value (C) of dV/dD in the range of 0.30 $\mu$m to 10.0 $\mu$m is 0.01 to 100.00, and a more preferred upper limit thereof is 50.00, 10.00, 5.00, 2.50, 1.20, 1.10, 1.00, 0.90, 0.80, and 0.70 in order, and particularly preferably 0.65. A lower limit thereof is 0.02 and 0.05 in order of further preference, and particularly preferably 0.06. Therefore, a more preferred range thereof is 0.01 to 50.00, 0.01 to 10.00, 0.01 to 5.00, 0.01 to 2.50, 0.01 to 1.20, 0.01 to 1.10, 0.01 to 1.00, 0.01 to 0.90, 0.02 to 0.80, and 0.05 to 0.70 in order, and a most preferred range thereof is 0.06 to 0.65.

A preferred range of a maximum value (D) of dV/dD in the range of the pore diameter of 0.040 $\mu$m to 0.30 $\mu$m is 0.01 to 100.00, and a more preferred upper limit thereof is 50.00, 10.00, 5.00, 2.50, 1.90, 1.50, 1.00, 0.75, and 0.50 in order, and particularly preferably 0.45. A lower limit thereof is 0.02, 0.05, 0.10, 0.15, 0.20, and 0.25 in order of further preference, and particularly preferably 0.30. Therefore, a more preferred range thereof is 0.01 to 50.00, 0.01 to 10.00, 0.01 to 5.00, 0.02 to 2.50, 0.05 to 1.90, 0.10 to 1.50, 0.15 to 1.00, 0.20 to 0.75, and 0.25 to 0.50 in order, and a most preferred range thereof is 0.30 to 0.45.

[(F/E)]

Preferably, the catalyst according to the present invention has at least one peak in a pore diameter range of 0.040 $\mu$m to 0.35 $\mu$m and has at least one peak in a pore diameter range of 0.35 $\mu$m to 100.0 $\mu$m in the differential pore volume distribution (dV vs D), and when a maximum value of dV in the pore diameter range of 0.35 $\mu$m to 100.0 $\mu$m is represented by E and a maximum value of dV in the pore diameter range of 0.040 $\mu$m to 0.35 $\mu$m is represented by F, (F/E) is 0.23 to 1,000.00. dV represents a mercury filling volume (unit: mL/g) per sample weight in each measurement section measured by mercury porosimetry.

An upper limit of (F/E) is 500.00, 100.00, 75.00, 50.00, 25.00, 10.00, 5.00, 4.50, 3.00, and 2.50 in order of preference, and particularly preferably 2.35. A lower limit thereof is 0.25, 0.30, 0.40, and 0.50 in order of preference, and particularly preferably 0.54. Therefore, a more preferred range of (F/E) is 0.23 to 500.00, 0.23 to 100.00, 0.23 to 75.00, 0.23 to 50.00, 0.23 to 25.00, 0.23 to 10.00, 0.25 to 5.00, 0.30 to 4.50, 0.40 to 3.00, and 0.50 to 2.50 in order, and a most preferred range thereof is 0.54 to 2.35.

A preferred range of the maximum value of dV (E) in the range of 0.35 $\mu$m to 100.0 $\mu$m is 0.0001 to 0.5000, and a more preferred upper limit thereof is 0.2500, 0.1000, 0.0500, 0.0250, 0.0200, 0.0150, 0.0100, 0.0050, 0.0045, and 0.0040 in order, and particularly preferably 0.0035. A lower limit thereof is 0.0002 and 0.0003 in order of further preference, and particularly preferably 0.0005. Therefore, the range thereof is 0.0001 to 0.2500, 0.0001 to 0.1000, 0.0001 to 0.0500, 0.0001 to 0.0250, 0.0001 to 0.0200, 0.0001 to 0.0150, 0.0001 to 0.0100, 0.0001 to 0.0050, 0.0002 to 0.0045, and 0.0003 to 0.0040 in order of more preference, and a most preferred range thereof is 0.0005 to 0.0035.

A preferred range of a maximum value of dV (F) in the range of the pore diameter of 0.040 $\mu$m to 0.35 $\mu$m is 0.0001 to 0.5000, and a more preferred upper limit thereof is 0.2500, 0.1000, 0.0500, 0.0250, 0.0200, 0.0150, 0.0100, 0.0050, 0.0040, and 0.0030 in order, and particularly preferably 0.0020. A lower limit thereof is 0.0002, 0.0005, 0.0008, and 0.0010 in order of further preference, and particularly preferably 0.0012. Therefore, the range is 0.0001 to 0.2500, 0.0001 to 0.1000, 0.0001 to 0.0500, 0.0001 to 0.0250, 0.0001 to 0.0200, 0.0001 to 0.0150, 0.0002 to 0.0100, 0.0005 to 0.0050, 0.0008 to 0.0040, 0.0010 to 0.0030 in order of more preference, and a most preferred range thereof is 0.0012 to 0.0020.

[Cumulative Specific Surface Area]

Preferably, the catalyst according to the present invention has a cumulative specific surface area of less than 5.0 $m^2/g$. In the related art, a catalyst having a cumulative specific surface area of less than 5.0 $m^2/g$ has a disadvantage in that the reaction rate decreases and the yield decreases, and it is considered that the feasibility is not high as long as there is no technique for compensating the disadvantage. On the other hand, it can be expected that the strength of the catalyst may increase when the catalyst has a small cumulative specific surface area, as described above. In the present invention, a specific numerical range has been adopted for the above (B/A), and this range has been implemented based on the composition and the production method. As a result, even less than 5.0 $m^2/g$ of the cumulative specific surface area has allowed for overcoming the disadvantage and making use of the advantage successfully.

A preferred upper limit of the cumulative specific surface area is 4.5 $m^2/g$, 4.0 $m^2/g$, 3.5 $m^2/g$, 3.3 $m^2/g$, 3.0 $m^2/g$, 2.7 $m^2/g$, and 2.5 $m^2/g$ in order, particularly preferably 2.0 $m^2/g$. A lower limit thereof can be assumed to be about 1.0 $m^2/g$, but a preferred lower limit is 1.1 $m^2/g$, 1.2 $m^2/g$, 1.3 $m^2/g$, and 1.4 $m^2/g$ in order, and particularly preferably 1.5 $m^2/g$. Therefore, a preferred range of the cumulative specific surface area is 1.1 $m^2/g$ to 4.5 $m^2/g$, 1.1 $m^2/g$ to 4.0 $m^2/g$, 1.1 $m^2/g$ to 3.5 $m^2/g$, 1.1 $m^2/g$ to 3.3 $m^2/g$, 1.2 $m^2/g$ to 3.0 $m^2/g$, 1.3 $m^2/g$ to 2.7 $m^2/g$, and 1.4 $m^2/g$ to 2.5 $m^2/g$ in order, and most preferably 1.5 $m^2/g$ to 2.0 $m^2/g$.

Examples of a method for changing a pore volume parameter include adjustment of pH in the blending step and control of the temperature in the drying step, but the particularly effective method is adjustment of pH in the blending step.

[Adjustment of pH]

That is, pH of the blended solution is adjusted to a range of 1.0 to 7.5 before charging a raw material for iron component, in the blending step of adding and mixing a supply source compound for molybdenum, a supply source compound for bismuth, and a supply source compound for iron to a solvent or a solution and heating the mixture to prepare a blended solution. Examples of the adjustment of pH include a method of lowering the pH by adding nitric acid or a method of raising the pH by adding ammonia water or the like, which will be described in detail later.

A lower limit of the pH of the blended solution is more preferably 1.5, and is 1.8, 2.0, 2.2, 2.5, 2.7, 3.0, 3.1, 3.2, 3.5, 3.7, 3.8, and 3.9 in order of further preference. When the pH of the blended solution is lower than 3.0, a precipitate or gel may be generated in the blended solution and may be clogged in a nozzle of a spray dryer in the drying step to be described later. However, controlling spray conditions and pH as in the present invention allows for change in a pore structure and for avoiding the problem of clogging of the spray. An upper limit of the pH of the blended solution is more preferably 7.3, and is 7.0, 6.7, 6.5, 6.3, 6.1, 6.0, 5.9, 5.8, 5.5, 5.3, 5.0, 4.8, and 4.5 in order of further preference. That is, a range of a value of the pH of the blended solution is 1.5 to 7.3, 1.5 to 7.0, 1.8 to 6.7, 2.0 to 6.5, 2.2 to 6.3, 2.5 to 6.1, 2.7 to 6.0, 3.0 to 5.9, 3.1 to 5.8, 3.2 to 5.5, 3.5 to 5.3, 3.7 to 5.0, and 3.8 to 4.8 in order of preference, and most preferably 3.9 to 4.5. In the present invention, the pH may fall within the above range even without adding a pH adjusting agent before charging the raw material for iron component, but the essence of the present invention is to adjust the pH within the above range by adding a pH adjusting agent before charging the raw material for iron component.

In the method of lowering the pH, examples of the pH adjusting agent include acids such as nitric acid, sulfuric acid, hydrochloric acid, and oxalic acid, which are typically used as catalyst raw materials by those skilled in the art for pH adjustment. The examples also include pH adjusting agents such as phosphoric acid, boric acid, molybdic acid, and iron nitrate, whose elements remain after calcination, as long as the elemental composition of the catalytically active component to be described later is not changed. Among them, nitric acid is most preferred. In the method of raising the pH, examples of the pH adjusting agent include ammonia water, pyridine, and an ammonium carbonate aqueous solution, which are typically used as catalyst raw materials by those skilled in the art for pH adjustment. The examples also include pH adjusting agents such as potassium hydroxide and cesium hydroxide, whose elements remain after calcination, as long as the elemental composition of the catalytically active component to be described later is not changed. Among them, ammonia water is most preferred.

In the case of performing this method, the most preferred embodiment is a method of preparing a slurry in the order of adding the raw material for iron component and then adding a bismuth raw material to the blended solution after stirring.

For dropwise adding of the pH adjusting agent, the pH adjusting agent is charged while stirring at a stirring power of 0.01 $kw/m^3$ to 5.00 $kw/m^3$. A lower limit of the stirring power is preferably 0.05 $kw/m^3$, 0.10 $kw/m^3$, 0.50 $kw/m^3$,

and 1.00 kw/m$^3$, and an upper limit of the stirring power is preferably 4.50 kw/m$^3$, 4.00 kw/m$^3$, 3.50 kw/m$^3$, and 3.00 kw/m$^3$. That is, a range of the stirring power is 0.05 kw/m$^3$ to 4.50 kw/m$^3$, 0.10 kw/m$^3$ to 4.00 kw/m$^3$, and 0.50 kw/m$^3$ to 3.50 kw/m$^3$ in order of preference, and most preferably 1.00 kw/m$^3$ to 3.00 kw/m$^3$.

A dropping time of the pH adjusting agent is 1 second to 5 minutes. A lower limit of the dropping time is preferably 5 seconds, 10 seconds, and 15 seconds, and an upper limit of the dropping time is preferably 4 minutes, 3 minutes, 2 minutes and 30 seconds, 2 minutes, 1 minute and 30 seconds, 1 minute, 45 seconds, and 30 seconds. That is, a range of the dropping time is 5 seconds to 4 minutes, 5 seconds to 3 minutes, 5 seconds to 2 minutes and 30 seconds, 5 seconds to 2 minutes, 5 seconds to 1 minute and 30 seconds, 5 seconds to 1 minute, and 10 seconds to 45 seconds in order of preference, and most preferably 15 seconds to 30 seconds.

A solution temperature of the blended solution at the time of dropwise adding the pH adjusting agent is 5°C to 80°C. A lower limit of the solution temperature is preferably 10°C, 20°C, and 30°C, and an upper limit of the solution temperature is preferably 70°C, 60°C, 50°C, and 40°C. That is, a range of the solution temperature is 10°C to 70°C, 10°C to 60°C, and 20°C to 50°C in order of preference, and most preferably 30°C to 40°C.

[Drying Conditions]

As described above, the pH is adjusted in the blending step as one of the methods for obtaining the catalyst according to the present invention. In addition, an effective method is to optimize a rotational speed of a sprayer (rotary atomizer) in the case of spray drying. The optimum rotational speed of the rotary atomizer is not unconditionally determined because it is affected by a structure of the rotary atomizer or the spray dryer, the temperature, the pH, and a viscosity of the liquid to be dried, a blending ratio of catalyst components, etc., but is preferably 10,000 rpm to 18,000 rpm. A further preferred upper limit of the rotational speed of the rotary atomizer is 17,000 rpm, particularly preferably 16,000 rpm, and most preferably 15,000 rpm. A further preferred lower limit thereof is 11,000 rpm, a particularly preferred lower limit thereof is 12,000 rpm, and a most preferred lower limit thereof is 13,000 rpm. That is, a more preferred range of the rotational speed of the rotary atomizer is 11,000 rpm to 17,000 rpm and 12,000 rpm to 16,000 rpm in order, and most preferably 13,000 rpm to 15,000 rpm.

The rotational speed of the rotary atomizer is also represented by relative centrifugal acceleration, and is preferably 6,000 G to 20,000G. A more preferred lower limit thereof is 7,500 G, 8,500 G, and 10,000 G in order, and a more preferred upper limit thereof is 18,000 G, 16,000 G, and 14,000 G in order. That is, a more preferred range of the rotational speed of the rotary atomizer is 7,500 G to 18,000 G, and 8,500 G to 16,000 G in order, and most preferably 10,000 G to 14,000 G.

An inlet temperature of the sprayer for spray drying is preferably 180°C to 320°C. A more preferred lower limit thereof is 200°C, 220°C, and 230°C in order, and a more preferred upper limit thereof is 300°C, 280°C, and 270°C in order. That is, a more preferred range of the inlet temperature is 200°C to 300°C, and 220°C to 280°C, and most preferably 230°C to 270°C.

An outlet temperature of the sprayer for spray drying is preferably 100°C to 150°C. A more preferred lower limit thereof is 101°C, 102°C, 103°C, 104°C, and 105°C in order, and a more preferred upper limit thereof is 140°C, 130°C, and 120°C in order. That is, a more preferred range of the outlet temperature is 101°C to 150°C, 102°C to 150°C, 103°C to 140°C, and 104°C to 130°C in order, and most preferably 105°C to 120°C.

A difference between the inlet temperature and the outlet temperature is preferably 30°C to 220°C. A more preferred lower limit thereof is 50°C, 80°C, 100°C, and 120°C in order, and a more preferred upper limit thereof is 200°C, 180°C, 165°C, and 150°C in order. That is, a more preferred range of the difference between the inlet temperature and the outlet temperature is 50°C to 200°C, 80°C to 180°C, and 100°C to 165°C in order, and most preferably 120°C to 150°C.

Further, a range of a parameter SD given by the following formula in the method for producing the catalyst according to the present invention is preferably 22 to 51. A more preferred lower limit thereof is 26, 28, 30, 32, 34, and 35 in order, and a more preferred upper limit thereof is 48, 44, 42, and 40 in order. That is, a more preferred range of SD is 26 to 51, 28 to 51, 30 to 48, 32 to 44, and 34 to 42, and most preferably 35 to 40.

SD = 51.3 + 0.0766 $\times$ {(inlet temperature of spray dryer, unit: °C) - (outlet temperature of spray dryer, unit: °C)} - 0.00173 $\times$ (rotational speed of atomizer, unit: rpm)

That is, the catalyst according to the present invention can be obtained by appropriately controlling the pH in the blending step and the drying conditions in the drying step. Further, the catalyst according to the present invention can also be obtained by combining other methods described below as necessary.

[Other Methods]

As a method for obtaining the catalyst according to the present invention, control can be performed by changing conditions in each production step to be described later, and examples thereof include (I) a method of changing the catalyst composition, (II) a method of changing calcination conditions, (III) a method of changing temperature-lowering

8

conditions after calcination, (IV) a method of performing controlling so as not to apply a mechanical strength to a catalyst and a precursor thereof in all steps of catalyst production, (V) a method of using a raw material having high purity, and a method of combining two or more methods among (I) to (VIII). Details of a method (VI), a method (VII), and a method (VIII) will be described later.

Regarding the method (I), in the composition formula (1) to be described later, an upper limit of $e_1/b_1$ is 4.00, 3.50, and 3.00 in order as desired, a lower limit of $e_1/b_1$ is 0.10, 0.50, 1.00, 1.40, 1.50, 1.70, 1.90, and 2.00 in order as desired, and a range of $e_1/b_1$ is 0.10 to 4.00, 0.50 to 4.00, 1.00 to 4.00, 1.40 to 4.00, 1.50 to 4.00, 1.70 to 4.00, 1.90 to 3.50, and 2.00 to 3.00 in order of preference. An upper limit of $d_1/b_1$ is 12.0, 11.0, 10.0, and 9.5 in order as desired, a lower limit of $d_1/b_1$ is 2.0, 3.0, 4.0, 5.0, 5.5, 6.0, 6.5, and 7.0 in order as desired, and a range of $d_1/b_1$ is 2.0 to 12.0, 3.0 to 12.0, 4.0 to 12.0, 5.0 to 12.0, 5.5 to 12.0, 6.0 to 11.0, 6.5 to 10.0, and 7.0 to 9.5 in order of preference. An upper limit of $c_1/e_1$ is 4.0, 3.0, 2.5, 2.0, and 1.7 in order as desired, a lower limit of $c_1/e_1$ is 0.1, 0.4, 0.6, and 0.8 in order as desired, and a range of $c_1/e_1$ is 0.1 to 4.0, 0.1 to 3.0, 0.4 to 2.5, 0.6 to 2.0, and 0.8 to 1.7 in order of preference. An upper limit of $c_1/d_1$ is 2.0, 1.0, 0.8, and 0.6 in order as desired, a lower limit of $c_1/d_1$ is 0.1 and 0.2 in order as desired, and a range of $c_1/d_1$ is 0.1 to 2.0, 0.1 to 1.0, 0.1 to 0.8, and 0.2 to 0.6 in order of preference. An upper limit of $g_1/d_1$ is 0.100, 0.050, 0.040, 0.030, 0.020, and 0.015 in order as desired, a lower limit of $g_1/d_1$ is 0.001, 0.002, 0.003, 0.004, and 0.005 in order of preference, and a range of $g_1/d_1$ is preferably 0.001 to 0.100, 0.001 to 0.050, 0.002 to 0.040, 0.003 to 0.030, 0.004 to 0.020, and 0.005 to 0.015 in order of preference. An upper limit of $g_1/c_1$ is 0.070, 0.050, and 0.046 in order as desired, a lower limit of $g_1/c_1$ is 0.005, 0.010, and 0.012 in order as desired, and a range of $g_1/c_1$ is 0.005 to 0.070, 0.010 to 0.050, and 0.012 to 0.046 in order of preference.

Regarding the method (II), a calcination temperature is 200°C to 600°C, preferably 300°C to 550°C, and more preferably 460°C to 550°C in the preliminary calcination, the main calcination, which will be described later, or both. A calcination time is 0.5 hours or longer, preferably 1 hour or longer and 40 hours or shorter, more preferably 2 hours or longer and 15 hours or shorter, and most preferably 2 hours or longer and 9 hours or shorter. A calcination atmosphere is at an oxygen concentration of 10 vol% to 40 vol%, preferably 15 vol% to 30 vol%, and is most preferably an air atmosphere.

Regarding the method (III), in the preliminary calcination, the main calcination, which will be described later, or both, a temperature decrease rate (temperature-lowering rate) on a catalyst surface from a maximum temperature (preliminary calcination temperature or main calcination temperature) during a calcination step to room temperature is 1 °C/min to 200 °C/min, preferably 5 °C/min to 150 °C/min, more preferably 10 °C/min to 120 °C/min, and most preferably 50 °C/min to 100 °C/min. Temperature lowering methods to be typically used industrially in order to implement the above temperature-lowering rate range, for example, a method of exposing a catalyst taken out from a firing furnace after calcination to an inert atmosphere or mist of an inert solvent, and a method of rapidly moving a catalyst after calcination into a sufficiently pre-cooled chamber, are all within the scope of the present invention.

The method (IV) is a method of performing controlling so as not to apply mechanical impact, shear stress, etc., to the catalyst precursor to be described later and/or granules formed in each step. In this method, the mechanical impact, shear stress, etc., that may be applied to the granules are controlled to 100 kgf or less, preferably 50 kgf or less, more preferably 20 kgf or less, further preferably 10 kgf or less, and most preferably 5 kgf or less.

Regarding the method (V), details are not limited as long as it is a method of using a reagent-grade high-purity raw material, and a raw material is used in which, for example, a total content of sulfur and a compound thereof, lithium, a halogen and compounds thereof, and lead is 10,000 ppm by weight or less, preferably 1,000 ppm by weight or less, more preferably 100 ppm by weight, and most preferably 10 ppm by weight or less.

Examples of the method (VI) include a method of once obtaining a catalyst precursor as granules to be described later and molding the granules. Obtaining the catalyst precursor as granules allows for production of catalysts in which each component of the catalyst is more uniformed.

The method (VII) is a method of controlling time during which a raw material for cobalt component and a raw material for nickel component are mixed, reacted, slurried, and retained in a blending vessel as short as possible in the blending step of the catalyst described later, and more specifically, is a method of shortening the residence time after mixing, in the blending vessel, an aqueous metal salt solution derived from molybdenum and alkali metals and an aqueous metal salt solution derived from cobalt and nickel, or a method of shortening the residence time after an aqueous metal salt solution derived from cobalt and nickel is charged into a blending vessel after the pH in the blending vessel is controlled to the specific range as described above. The residence time is preferably 24 hours, more preferably 1 hour, further preferably 30 minutes, and most preferably 10 minutes. The range of the pH is 1 to 14, preferably 2 to 10, more preferably 2 to 8, and most preferably 3 to 7.

Examples of the method (VIII) include a method of charging each raw material at once in the blending step undividedly or a method of reducing a nitric acid concentration in the blended solution, in the blending step to be described later. The above method of charging at once means charging a next raw material after all the required amount of a raw material is charged. Regarding a method of reducing the nitric acid concentration in the blended solution, a nitrate ion concentration in terms of mass% in a blended solution at the time when the blending is completed and ready to proceed to the next step is preferably 40 mass% or less, more preferably 35 mass% or less, further preferably 30 mass% or less, and most preferably 25 mass% or less.

[Composition]

The catalyst according to the present invention preferably contains molybdenum, bismuth, and iron, and more preferably has a composition represented by the following formula (1).

$$Mo_{a1}Bi_{b1}Ni_{c1}CO_{d1}Fe_{e1}X_{f1}Y_{g1}Z_{h1}O_{i1} \qquad (1)$$

(In the formula, Mo, Bi, Ni, Co, and Fe respectively represent molybdenum, bismuth, nickel, cobalt, and iron; X is at least one element selected from tungsten, antimony, tin, zinc, chromium, manganese, magnesium, calcium, silicon, aluminum, cerium, and titanium; Y is at least one element selected from sodium, potassium, cesium, rubidium, and thallium; Z belongs to Groups 1 to 16 in the periodic table, and means at least one element selected from elements other than the above Mo, Bi, Ni, Co, Fe, X, and Y; $a1$, $b1$, $c1$, $d1$, $e1$, $f1$, $g1$, $h1$, and $i1$ respectively represent the number of atoms of molybdenum, bismuth, nickel, cobalt, iron, X, Y, Z, and oxygen; and when $a1 = 12$, $0 < b1 \le 7$, $0 \le c1 \le 10$, $0 < d1 \le 10$, $0 < c1+d1 \le 20$, $0 < e1 \le 5$, $0 \le f1 \le 2$, $0 \le g1 \le 3$, $0 \le h1 \le 5$, and $i1$ is a value determined by an oxidation state of each element).
In the above formula (1), when $a1 = 12$, preferred ranges of $b1$ to $h1$ are as follows.
A lower limit of $b1$ is 0.1, 0.2, 0.3, 0.4, 0.5, and 0.6 in order of preference, and an upper limit thereof is 6.0, 5.0, 4.0, 3.0, 2.0, 1.8, 1.5, 1.2, 1.0, and 0.9 in order of preference. That is, a range of $b1$ is 0.1 to 6.0, 0.1 to 5.0, 0.1 to 4.0, 0.1 to 3.0, 0.1 to 2.0, 0.2 to 1.8, 0.3 to 1.5, 0.4 to 1.2, 0.5 to 1.0 in order of preference, and a most preferred range thereof is 0.6 to 0.9.
A lower limit of $c1$ is 0.1, 0.2, 0.3, 0.5, 0.7, 0.9, 1.1, 1.3, and 1.5 in order of preference, and an upper limit thereof is 8.0, 7.0, 6.0, 5.0, 4.0, 3.5, and 3.3 in order of preference. That is, a range of $c1$ is 0.1 to 8.0, 0.2 to 8.0, 0.3 to 8.0, 0.5 to 8.0, 0.7 to 8.0, 0.8 to 7.0, 0.9 to 6.0, 1.0 to 5.0, 1.1 to 4.0, and 1.3 to 3.5 in order of preference, and a most preferred range thereof is 1.5 to 3.3.
A lower limit of $d1$ is 1.0, 2.0, 3.0, 4.0, 5.0, and 5.4 in order of preference, and an upper limit thereof is 10.0, 9.5, 9.0, 8.5, 8.0, 7.5, and 7.0 in order of preference. That is, a range of $d1$ is 1.0 to 10.0, 1.0 to 9.5, 2.0 to 9.0, 3.0 to 8.5, 4.0 to 8.0, and 5.0 to 7.5 in order of preference, and a most preferred range thereof is 5.4 to 7.0.
A lower limit of $c1+d1$ is 1.1, 2.0, 4.0, 6.0, 7.0, and 7.8 in order of preference, and an upper limit thereof is 20.0, 15.0, 12.5, 11.0, 10.0, and 9.5 in order of preference. That is, a range of $c1+d1$ is 1.1 to 20.0, 2.0 to 15.0, 4.0 to 12.5, 6.0 to 11.0, and 7.0 to 10.0 in order of preference, and a most preferred range thereof is 7.8 to 9.5.
A lower limit of $e1$ is 0.1, 0.2, 0.5, 0.8, 1.0, 1.5, 1.6, and 1.7 in order of preference, and an upper limit thereof is 5.0, 4.5, 4.0, 3.5, 3.0, 2.5, and 2.2 in order of preference. That is, a range of $e1$ is 0.1 to 5.0, 0.2 to 5.0, 0.5 to 4.5, 0.8 to 4.0, 1.0 to 3.5, 1.5 to 3.0, and 1.6 to 2.5 in order of preference, and a most preferred range thereof is 1.7 to 2.2.
An upper limit of $f1$ is 1.8, 1.5, 1.0, 0.8, and 0.5 in order of preference, and a lower limit thereof is preferably 0. That is, a range of $f1$ is 0 to 1.8, 0 to 1.5, 0 to 1.0, 0 to 0.8, and 0 to 0.5 in order of preference, and $f1$ is most preferably 0.
A lower limit of $g1$ is 0.01, 0.02, and 0.03 in order of preference, and an upper limit thereof is 2, 1, 0.5, 0.4, 0.3, 0.2, 0.15, and 0.09 in order of preference. That is, a range of $g1$ is 0.01 to 2, 0.01 to 1, 0.01 to 0.5, 0.01 to 0.4, 0.01 to 0.3, 0.01 to 0.2, and 0.02 to 0.15 in order of preference, and a most preferred range thereof is 0.03 to 0.09.
An upper limit of $h1$ is 4.0, 3.0, 2.0, 1.8, 1.5, 1.0, 0.8, and 0.5 in order of preference, and a lower limit thereof is preferably 0. That is, a range of $h1$ is 0 to 4.0, 0 to 3.0, 0 to 2.0, 0 to 1.8, 0 to 1.5, 0 to 1.0, 0 to 0.8, and 0 to 0.5 in order of preference, and $h1$ is most preferably 0.
X in the formula (1) is preferably tungsten, antimony, zinc, magnesium, calcium, or cerium, and particularly preferably antimony or zinc.
Y in the formula (1) is preferably sodium, potassium, or cesium, more preferably potassium or cesium, and particularly preferably potassium.
Z in the formula (1) is preferably phosphorus.
A drying temperature is not limited as long as moisture can be removed, and may be room temperature (25°C) when the pressure or time is adjusted. However, in order to more reliably remove moisture in a short period of time, the drying temperature is preferably 80°C or higher, and more preferably 90°C or higher. When the pressure is not adjusted, the drying temperature is preferably 100°C or higher, and more preferably 150°C or higher.
In the preliminary calcination, calcination is performed at a temperature of about 200°C to 600°C for about 1 hour to 12 hours. An atmosphere and a heating rate at the time of calcination are not limited as long as they are in the range known by those skilled in the art. For example, the atmosphere is an air atmosphere, an inert atmosphere such as nitrogen, or an organic compound-containing atmosphere containing more than 0% of an organic compound such as methanol or ethanol, and the heating rate is 0.01 °C/min to 10 °C/min.

[Carrier]

A catalyst in which a preliminary calcined powder obtained by preliminary calcination after preparation of the catalyst is carried on an inert carrier is particularly effective as the catalyst according to the present invention.

As a material of the inert carrier, known substances such as alumina, silica, titania, zirconia, niobia, silica alumina, silicon carbide, carbides, and mixtures thereof can be used. Further, the particle diameter, water absorption, the mechanical strength, crystallinity of each crystal phase, and a mixing proportion are not limited, and an appropriate range can be selected in consideration of the final catalyst performance, molding properties, production efficiency, and the like. A mixing proportion of the carrier to the preliminary calcined powder is calculated as an active mass ratio based on the charged mass of each raw material according to the following equation. When a catalyst molding aid, a strength improver, or the like is used in the present operation, the content of these is included in the total amount (denominator).

Active mass ratio (mass%) = (mass of preliminary calcined powder for use in molding)/{(mass of preliminary calcined powder for use in molding) + (mass of carrier for use in molding)] $\times$ 100

A preferred upper limit of the active mass ratio is 80 mass%, and further preferably 60 mass%.

In addition, a preferred lower limit thereof is 20 mass%, and further preferably 30 mass%. That is, a preferred range of the active mass ratio is 20 mass% to 80 mass%, and most preferably 30 mass% to 60 mass%.

The inert carrier is preferably silica and/or alumina, and particularly preferably a mixture of silica and alumina.

A binder is preferably used for carrying. Specific examples of the binder to be used include water, ethanol, methanol, propanol, a polyhydric alcohol, polyvinyl alcohol as a polymer-based binder, and a silica sol aqueous solution as an inorganic binder. Ethanol, methanol, propanol, and a polyhydric alcohol are preferred, a diol such as ethylene glycol and a triol such as glycerin are preferred, and an aqueous solution having a concentration of glycerin of 5 mass% or more is preferred. Using an appropriate amount of a glycerin aqueous solution allows for providing a high-performance catalyst having good molding properties and high mechanical strength. An amount of these binders to be used is typically 2 parts by mass to 60 parts by mass with respect to 100 parts by mass of the preliminary calcined powder, and 10 parts by mass to 30 parts by mass is preferred in the case of a glycerin aqueous solution. The binder and the preliminary calcined powder may be alternately supplied to a molding machine or simultaneously supplied to the molding machine during carrying.

[Method for Producing Catalyst, etc.]

Starting raw materials for the elements constituting the catalyst according to the present invention are not limited, but for example, as the raw material for molybdenum component, molybdenum oxides such as molybdenum trioxide, molybdic acid or salts thereof, such as molybdic acid, ammonium paramolybdate, and ammonium metamolybdate, and heteropoly acids containing molybdenum or salts thereof, such as phosphomolybdic acid and silicomolybdic acid, can be used.

As the raw material for bismuth component, bismuth salts such as bismuth nitrate, bismuth carbonate, bismuth sulfate, and bismuth acetate, bismuth trioxide, metal bismuth, etc., can be used. These raw materials can be used as solids or as an aqueous solution, a nitric acid solution, or a slurry of bismuth compounds generated from these aqueous solutions. A nitrate, a solution thereof, or a slurry obtained from the solution is preferably used.

As starting raw materials for other component elements, an ammonium salt, a nitrate, a nitrite, a carbonate, a subcarbonate, an acetate, a chloride, an inorganic acid, an inorganic acid salt, a heteropoly acid, a heteropoly acid salt, a sulfate, a hydroxide, an organic acid salt, and an oxide of metallic elements that are typically used in this type of catalyst, or a mixture thereof may be used in combination. An ammonium salt and a nitrate are preferably used.

A compound containing these active components may be used alone or in combination of two or more thereof. A slurry liquid can be obtained by uniformly mixing each active component-containing compound and water. An amount of water to be used in the slurry liquid is not limited as long as it can completely dissolve the entire amount of the compound used or enables uniform mixing. The amount of water to be used may be appropriately determined in consideration of the drying method and drying conditions. The amount of water to be used is usually 100 parts by mass to 2,000 parts by mass with respect to 100 parts by mass of the total mass of the compounds for preparing slurry. The amount of water may be large, but too large amount of water has many disadvantages that the energy cost in the drying step increases, drying cannot be completed, and the like.

The slurry liquid of the supply source compound for each component element is preferably prepared by (i) a method of mixing at once, (ii) a method of mixing at once and then performing aging, (iii) a method of mixing in a stepwise manner, (iv) a method of repeating a mixing step and an aging step in a stepwise manner, and a method combining two or more methods among (i) to (iv). Here, the above aging means "an operation in which industrial raw materials or semi-finished products are treated under specific conditions such as a certain period of time and a certain temperature to acquire or improve the required physical properties and chemical properties, or proceed with a predetermined reaction". In the present invention, the above certain period of time means a range of 5 minutes or longer and 24 hours or shorter, and the above certain temperature means a range equal to or higher than room temperature and equal to or lower than a boiling point of an aqueous solution or an aqueous dispersion liquid. Among these, in terms of the activity and the yield of the finally obtained catalyst, preferred is (iii) the method of mixing in a stepwise manner, more preferred is a method of preparing a completely dissolved solution for each raw material to be mixed with a mother liquid in a stepwise manner, and most preferred is a method of mixing various mixed solutions of an alkali metal solution and a nitrate with a mother liquid containing a raw material for molybdenum component in a form of a blended solution or slurry. However, it is not always necessary to mix all the catalyst constituent elements in this step, and some elements or some amounts thereof may be added in the subsequent steps.

In the present invention, a shape of stirring blades of a stirrer to be used in mixing essential active component is not limited, and any stirring blades such propeller blades, turbine blades, paddle blades, inclined paddle blades, screw blades, anchor blades, ribbon blades, and large lattice blades can be used in one stage or two or more stages of the same type of blade or different types of blades in an up-down direction. In addition, a baffle (baffle plate) may be installed in a reaction vessel as required.

Next, the slurry liquid thus obtained is dried. The drying method is not limited as long as it is a method capable of completely drying the slurry liquid, and examples thereof include drum drying, freeze drying, spray drying, and evaporation to dryness. Among these, in the present invention, spray drying is particularly preferred because the slurry liquid can be dried into a powder or granules in a short period of time. A drying temperature in the spray drying varies depending on the concentration of the slurry liquid, the liquid feeding rate, or the like, and the temperature at an outlet of a dryer is typically 70°C to 150°C.

The catalyst precursor obtained as described above is preliminary calcined, shaped, and then subjected to main calcination. This allows for controlling and keeping a formed shape of a catalyst to provide a catalyst particularly excellent in mechanical strength for industrial use, and the catalyst achieves stable catalyst performance.

As the molding, both molding methods of carrying-molding of carrying on a carrier such as silica and non-carrying-molding without using a carrier can be used. Specific molding methods include tablet molding, press molding, extrusion molding, and granulation molding. As a shape of a molded product, for example, a columnar shape, a ring shape, a spherical shape, or the like may be appropriately selected in consideration of operating conditions. Preferred is a carried catalyst in which a catalyst precursor is carried on a spherical carrier, particularly an inert carrier such as silica or alumina, having an average particle diameter of 3.0 mm to 10.0 mm, and preferably having an average particle diameter of 3.0 mm to 8.0 mm. As for a carrying method, a tumbling granulation, a method using a centrifugal flow coating apparatus, a wash coating and the like, are widely known. The method is not limited as long as the preliminary calcined powder can be uniformly carried on the carrier. The tumbling granulation is preferred in consideration of the production efficiency of the catalyst, and the like. Specifically, the tumbling granulation is a method of rotating a flat or uneven disc at a high speed in an apparatus that includes the disc at a bottom portion of a fixed cylindrical container, to vigorously stir a carrier charged in the container by repeating the rotation and revolution movements of the carrier, and carrying the powder component on the carrier by adding the preliminary calcined powder to the container. A binder is preferably used for carrying. Specific examples of the binder which can be used include water, ethanol, methanol, propanol, a polyhydric alcohol, a polyvinyl alcohol that is a polymer-based binder, and an aqueous silica sol solution that is an inorganic binder. Ethanol, methanol, propanol, and a polyhydric alcohol are preferred, a diol such as ethylene glycol and a triol such as glycerin are more preferred, and an aqueous solution having a concentration of glycerin of 5 mass% or more is further preferred. Using an appropriate amount of a glycerin aqueous solution allows for providing a high-performance catalyst having good molding properties and high mechanical strength. An amount of these binders to be used is typically 2 parts by mass to 60 parts by mass with respect to 100 parts by mass of the preliminary calcined powder, and 15 parts by mass to 50 parts by mass is preferred in the case of using a glycerin aqueous solution. The binder and the preliminary calcined powder may be alternately supplied to a molding machine or simultaneously supplied to the molding machine during carrying. Further, in the molding, a small amount of known additives such as graphite and talc may be added. None of a molding aid, a pore-forming agent, and a carrier added during the molding is considered as the constituent element of the active component in the present invention, regardless of presence or absence of activity in the sense of converting the raw material into some other product.

The method of preliminary calcination, the preliminary calcination conditions, the method of main calcination, and the main calcination conditions are not limited, and known treatment methods and conditions can be applied. The preliminary calcination or the main calcination is typically carried out at 200°C to 600°C, and preferably 300°C to 550°C, for 0.5 hours or longer, and preferably 1 hour or longer and 40 hours or shorter under a flow of an oxygen-containing gas such as air or a flow of an inert gas. Here, the inert gas refers to a gas that does not reduce the reaction activity of the catalyst, and specific examples thereof include nitrogen, carbon dioxide, helium, and argon. The optimum conditions for particularly the main calcination vary depending on the reaction conditions at the time of producing an unsaturated aldehyde and/or an unsaturated carboxylic acid using a catalyst, and it is known to those skilled in the art to change step parameters of the main calcination step, that is, the oxygen content in the atmosphere, the maximum temperature and the calcination time. Thus, the above change falls within the scope of the present invention. In addition, the main calcination step is carried out after the above preliminary calcination step, and the maximum temperature (main calcination temperature) in the main calcination step is higher than the maximum temperature (preliminary calcination temperature) in the above preliminary calcination step. The calcination method is not limited to a fluidized bed, a rotary kiln, a muffle furnace, a tunnel firing furnace, and the like, and an appropriate range should be selected in consideration of the final catalyst performance, the mechanical strength, the molding properties, the production efficiency, and the like.

The catalyst according to the present invention is preferably used as a catalyst for producing an unsaturated aldehyde compound, an unsaturated carboxylic acid compound, or a conjugated diene compound, is further preferably used as a catalyst for producing, more preferably, an unsaturated aldehyde compound, and is particularly preferably used as a catalyst for producing acrolein from propylene. In an exothermic reaction process such as the production of an unsaturated aldehyde compound, an unsaturated carboxylic acid compound, or a conjugated diene compound, it is known to those skilled in the art to fill different kinds of catalysts in multiple layers for increasing the activity from an inlet side of a reaction tube to an outlet side of the reaction tube, for the purpose of preventing deterioration of the catalyst itself due to the heat generated by the reaction in an actual plant. The catalyst according to the present invention can be used on one of the inlet side of the reaction tube, the outlet side of the reaction tube, and an intermediate catalyst layer, and is most preferably used, for example, on the most outlet side of the reaction tube, that is, used for the most active catalyst among all catalyst layers in the reaction tube. In multi-layer filling, two-layer or three-layer filling is a particularly preferred embodiment.

[Second-Stage Catalyst]

The catalyst according to the present invention can be used not only as a first-stage catalyst, that is, a catalyst for producing an unsaturated aldehyde compound, but also as a second-stage catalyst, that is, a catalyst for producing an unsaturated carboxylic acid compound.

As the second-stage catalyst, a catalyst represented by the following formula (2) is preferably used.

$$Mo_{12}V_{a2}W_{b2}Cu_{c2}Sb_{d2}X2_{e2}Y2_{f2}Z2_{g2}O_{h2} \qquad (2)$$

(In the formula, Mo, V, W, Cu, Sb, and O represent molybdenum, vanadium, tungsten, copper, antimony, and oxygen, respectively; X2 represents at least one element selected from the group consisting of an alkali metal and thallium; Y2 represents at least one element selected from the group consisting of magnesium, calcium, strontium, barium, and zinc; and Z2 represents at least one element selected from the group consisting of niobium, cerium, tin, chromium, manganese, iron, cobalt, samarium, germanium, titanium, and arsenic. In addition, a2, b2, c2, d2, e2, f2, g2, and h2 represent the atomic ratio of each element, and satisfy $0 < a2 \leq 10$, $0 \leq b2 \leq 10$, $0 < c2 \leq 6$, $0 < d2 \leq 10$, $0 \leq e2 \leq 0.5$, $0 \leq f2 \leq 1$, and $0 \leq g2 < 6$. Further, h2 is the number of oxygen atoms required to satisfy the valence of each component described above.).

In the production of the catalyst represented by the above formula (2), a method typically known as a method for preparing a catalyst of this type, for example, an oxide catalyst or a catalyst having a heteropoly acid or a salt structure thereof, can be adopted. When the catalyst according to the present invention is used as the second-stage catalyst, the above method for obtaining the catalyst according to the present invention can be appropriately applied in the production of the catalyst. The raw materials that can be used at the time of producing the catalyst are not limited, and various materials can be used. For example, molybdenum oxides such as molybdenum trioxide, molybdic acid or salts thereof such as molybdic acid and ammonium molybdate, molybdenum-containing heteropoly acids such as phosphomolybdic acid and silicomolybdic acid or salts thereof can be used. The antimony component raw material is not limited, and antimony trioxide or antimony acetate is preferred. As raw materials for other elements such as vanadium, tungsten, and copper, a nitrate, a sulfate, a carbonate, a phosphate, an organic acid salt, a halide, a hydroxide, and an oxide, or the metal can be used.

A compound containing these active components may be used alone or in combination of two or more thereof.

The slurry liquid obtained by mixing the above raw materials in water is dried to obtain a catalyst precursor solid. The drying method is not limited as long as it is a method capable of completely drying the slurry liquid, and examples thereof include drum drying, freeze drying, spray drying, and evaporation to dryness. Spray drying is preferred because the slurry liquid can be dried into a powder or granule in a short period of time. The drying temperature in the spray drying varies depending on the concentration of the slurry liquid, the liquid feeding rate, etc., but generally, a temperature at an inlet of the dryer is 140°C to 400°C, a temperature at an outlet is 70°C to 150°C, and the inlet temperature becomes higher than the outlet temperature. Further, it is preferable to perform drying such that an average particle diameter of a slurry liquid dried body obtained at this time is 10 μm to 700 μm.

A second-stage catalyst precursor solid obtained as described above can be directly supplied to a coating mixture, and is preferably subjected to calcination because the molding properties may be improved due to the calcination. The calcination method and the calcination conditions are not limited, and known treatment methods and conditions can be applied. Optimum calcination conditions vary depending on the catalyst raw material used, the catalyst composition, the preparation method, etc., and the calcination temperature is typically 100°C to 350°C, preferably 150°C to 300°C, and the calcination time is 1 hour to 20 hours. The calcination is typically carried out in an air atmosphere, and may be carried out in an atmosphere of an inert gas such as nitrogen, carbon dioxide, helium, or argon, or the calcination may be carried out in an air atmosphere after calcination in an inert gas atmosphere, if necessary. The calcined solid thus obtained is preferably pulverized before molding. A pulverization method is not limited, and it is preferable to use a ball mill.

The compound containing the active component at the time of preparing the second-stage slurry does not necessarily contain all the active components, and some of the components may be used before the following molding step.

The shape of the second-stage catalyst is not limited, and the catalyst is shaped into a columnar shape, a tablet, a ring shape, a spherical shape, or the like in order to reduce a pressure loss of a reaction gas in an oxidation reaction. Among these, the catalyst precursor solid is particularly preferably carried on an inert carrier to be a carried catalyst, as improvement in selectivity and removal of reaction heat can be expected. A tumbling granulation described below is preferred for the carrying. The tumbling granulation is a method of rotating a flat or uneven disc at a high speed in an apparatus that includes the disc at, for example, a bottom portion in a fixed container, to vigorously stir a carrier in the container by repeating rotation and revolution movements, and carrying, on the carrier, a mixture for carrying in which a binder, a catalyst precursor solid, and, if necessary, other additives such as a molding aid and a strength improver are added. As a method for adding the binder, any method may be used, such as 1) pre-mixing the binder with the mixture for carrying, 2) adding the binder simultaneously at the time of adding the mixture for carrying into the fixed container, 3) adding the binder after adding the mixture for carrying into the fixed container, 4) adding the binder before adding the mixture for carrying into the fixed container, 5) separating each of the mixture for carrying and the binder, and adding the total amount thereof by appropriately combining two or more methods among 2) to 4). Among these, in 5), it is preferable to adjust an addition rate by using an auto feeder or the like such that a predetermined amount of the mixture for carrying is carried on the carrier without, for example, the mixture for carrying adhering to walls of the fixed container or agglomeration of the mixture for carrying. Examples of the binder include water, ethanol, polyhydric alcohol, polyvinyl alcohol as a polymer-based binder, a cellulose such as crystalline cellulose, methyl cellulose, and ethyl cellulose, and an aqueous silica sol solution as an inorganic binder. A cellulose, a diol such as ethylene glycol, and a triol such as glycerin are preferred, and an aqueous solution having a concentration of glycerin of 5 mass% or more is particularly preferred. The amount of the binder to be used is typically 2 parts by mass to 60 parts by mass, and preferably 10 parts by mass to 50 parts by mass with respect to 100 parts by mass of the mixture for carrying.

Specific examples of the carrier in the above carrying include a spherical carrier having a diameter of 1 mm to 15 mm, and preferably 2.5 mm to 10 mm, such as silicon carbide, alumina, silica alumina, mullite, and alundum. As the carrier, those having a porosity of 10% to 70% are typically used. A proportion of the carrier and the mixture for carrying, mixture for carrying/(mixture for carrying + carrier) is typically 10 mass% to 75 mass%, and preferably 15 mass% to 60 mass%. When the proportion of the mixture for carrying is large, the reaction activity of the carried catalyst increases, but the mechanical strength tends to decrease. Conversely, when the proportion of the mixture for carrying is small, the mechanical strength increases, but the reaction activity tends to decrease. Examples of the molding aid to be used if necessary in the above include silica gel, diatomaceous earth, and an alumina powder. An amount of the molding aid to be used is typically 1 part by mass to 60 parts by mass with respect to 100 parts by mass of the catalyst precursor solid. In addition, if necessary, the use of inorganic fibers (for example, ceramic fibers or whiskers) inert to the catalyst precursor solid and the reaction gas, as the strength improver, is useful for improving the mechanical strength of the catalyst, and a glass fiber is preferred. An amount of the fiber to be used is typically 1 part by mass to 30 parts by mass with respect to 100 parts by mass of the catalyst precursor solid. None of the molding aid, the pore-forming agent, and the carrier added during the molding of the first-stage catalyst is not considered as the constituent element of the active component in the present invention, regardless of presence or absence of activity in the sense of converting the raw material into some other product.

The carried catalyst obtained as described above can be directly supplied as a catalyst for the gas-phase catalytic oxidation reaction, but is preferably subjected to calcination because the catalytic activity may be improved due to the calcination. The calcination method and the calcination conditions are not limited, and known treatment methods and conditions can be applied. The optimum calcination conditions vary depending on the catalyst raw material used, the catalyst composition, the preparation method, and the like, and the calcination temperature is typically 100°C to 450°C, preferably 270°C to 420°C, and the calcination time is 1 hour to 20 hours. The calcination is typically carried out in an air atmosphere, and may be carried out in an atmosphere of an inert gas such as nitrogen, carbon dioxide, helium, or argon, or the calcination may be carried out in an air atmosphere after calcination in an inert gas atmosphere, if necessary.

[Use of Catalyst, etc.]

The use of the catalyst according to the present invention in a reaction that uses propylene, isobutylene, t-butyl alcohol, or the like as a raw material to produce the corresponding unsaturated aldehyde and unsaturated carboxylic acid, particularly, in a reaction of producing acrolein and acrylic acid by gas-phase catalytic oxidation of propylene with molecular oxygen or a molecular oxygen-containing gas, allows for improving the catalytic activity and the yield, and it is very effective in improving the price competitiveness of the product as compared with the known method. In addition, the catalyst is also useful even when the catalyst is used as an oxidation catalyst or an oxidation dehydration catalyst in the case of producing a conjugated diolefin (such as 1,3-butadiene) by a catalytic oxidation dehydration reaction from a mixed gas containing molecular oxygen and a monoolefin (such as butenes) having four or more carbon atoms.

The use of the catalyst according to the present invention may achieve a long-term operation of a gas-phase catalytic oxidation method safely, stably, and at low cost. In addition, the catalyst according to the present invention may be effectively used for improvement in the yield even in a region where the catalytic activity is particularly high or a region where the catalytic activity is not high, and improvement in the process stability of a partial oxidation reaction accompanied by heat generation such as reduction of $\Delta T$ (difference between hotspot temperature and salt bath temperature (reaction temperature)) can also be expected. Further, the catalyst according to the present invention may also be effective in reducing by-products that adversely influence the environment and the quality of the final product, such as carbon monoxide (CO), carbon dioxide ($CO_2$), acetaldehyde, acetic acid, and formaldehyde.

The catalyst according to the present invention thus obtained can be used, for example, in producing acrolein and/or acrylic acid by catalytic gas phase oxidation of propylene by using a molecular oxygen-containing gas. In the production method according to the present invention, a method for distributing a raw material gas may be an ordinary single distribution method or a recycling method, can be carried out under typically used conditions, and is not limited. A mixed gas containing, as starting raw materials, for example, 1 vol% to 10 vol% and preferably 4 vol% to 9 vol% of propylene, 3 vol% to 20 vol% and preferably 4 vol% to 18 vol% of molecular oxygen, 0 vol% to 60 vol% and preferably 4 vol% to 50 vol% of water vapor, and 20 vol% to 80 vol% and preferably 30 vol% to 60 vol% of an inert gas such as carbon dioxide and nitrogen at room temperature is introduced, at 250°C to 450°C under normal pressure to 10 atm and a space velocity of 300 h$^{-1}$ to 5000 h$^{-1}$, into the catalyst according to the present invention filled in the reaction tube, and the reaction is carried out.

In the present description, unless otherwise specified, the improvement of the catalytic activity means that the raw material conversion rate is high when the catalytic reaction is carried out at the same salt bath temperature and comparison is made.

In the present invention, unless otherwise specified, a high yield means a high total yield of the corresponding unsaturated aldehyde and/or unsaturated carboxylic acid when the oxidation reaction is carried out using, or the like as a raw material. Unless otherwise specified, the yield refers to a useful yield to be described later.

In the present description, unless otherwise specified, the constituent elements of the catalyst precursor refer to all the elements used in the catalyst production step, but raw materials and constituent elements thereof that disappear, sublimate, volatilize, and burn at the maximum temperature or lower in the main calcination step are not included in the constituent elements of the active component of the catalyst. In addition, silicon contained in the molding aid and the carrier and other elements constituting inorganic materials in the molding step are not included as the constituent elements of the active component of the catalyst.

In the present description, the hotspot temperature is the maximum temperature in a temperature distribution in a catalyst-filled layer that is measured by installing thermocouples in a long axis direction in a multi-tube reaction tube, and the salt bath temperature is a set temperature of a heat medium used for the purpose of cooling the heat generation of the reaction tube. Although the number of points for measuring the temperature distribution is not limited, for example, the temperatures at 10 points to 1,000 points that equally divide a catalyst filling length are measured.

In the present description, the unsaturated aldehyde and the unsaturated aldehyde compound are organic compounds having at least one double bond and at least one aldehyde in the molecule, such as acrolein and methacrolein. In the present invention, the unsaturated carboxylic acid and the unsaturated carboxylic acid compound are organic compounds having at least one double bond and at least one carboxy group or an ester group thereof in the molecule, such as acrylic acid, methacrylic acid, and methyl methacrylate. In the present description, the conjugated diene is a chemically conjugated diene in which double bonds are separated by one single bond, such as 1,3-butadiene.

In the present description, the cumulative specific surface area is the sum of the specific surface areas in a specific pore diameter range of the catalyst measured by the above measurement method, and is the sum of the specific surface areas of the catalyst in a total pore diameter range that can be detected by measurement unless otherwise specified. In addition, it is also simply referred to as a specific surface area.

In the present description, the cumulative pore volume is the sum of the pore volumes of the catalyst in a specific pore diameter range measured by the above measurement method, and is the sum of the pore volumes of the catalyst in the total pore diameter range that can be detected by measurement unless otherwise specified. In addition, it is also simply referred to as a pore volume.

In the present invention, the pore volume distribution is a curve illustrating a distribution of a pore volume ratio at each pore diameter D, where a vertical axis represents -dV/d (log D) obtained by differentiating V by the natural logarithm of D and using the reciprocal number, and a horizontal axis represents D, when V represents the volume (unit: mL/g) of the filled mercury and D represents the pore diameter (unit: $\mu$m), in the measurement of the cumulative pore volume described above. The pore volume distribution is also described as Log differential pore diameter distribution, pore diameter distribution, or simply pore distribution.

EXAMPLES

Hereinafter, the present invention will be described in more detail with reference to Examples. In Examples, the conversion rate, the useful yield, the useful selectivity, and the active mass ratio were calculated according to the following equations.

Raw material conversion rate (%) = (number of moles of propylene reacted)/(number of moles of propylene supplied) $\times$ 100

Useful yield (%) = (total number of moles of acrolein and acrylic acid produced)/(number of moles of propylene supplied) $\times$ 100

Useful selectivity (%) = (total number of moles of acrolein and acrylic acid produced)/(number of moles of propylene reacted) $\times$ 100

Active mass ratio (mass%) = (mass of preliminary calcined powder for use in molding)/{(mass of preliminary calcined powder for use in molding) + (mass of carrier for use in molding)] $\times$ 100

[Example 1]

In 380 parts by mass of pure water heated to 60°C, 100 parts by mass of ammonium heptamolybdate was completely dissolved (mother liquid 1). Next, 0.32 parts by mass of potassium nitrate was dissolved in 2.9 parts by mass of pure water, and the solution was added to the mother liquid 1. Next, 10.6 parts by mass of nitric acid (60 mass%) was added to the mother liquid 1 to adjust the pH to 4. Next, 32 parts by mass of ferric nitrate, 82 parts by mass of cobalt nitrate, and 38 parts by mass of nickel nitrate were dissolved in 81 parts by mass of pure water heated to 60°C, and the solution was added to the mother liquid 1. Subsequently, 23 parts by mass of bismuth nitrate was dissolved in a nitric acid aqueous solution prepared by adding 5.8 parts by mass of nitric acid (60 mass%) to 24 parts by mass of pure water heated to 60°C, and the solution was added to the mother liquid 1. The mother liquid 1 was dried by spray drying using a rotary atomizer under conditions of 15,000 rpm, an inlet temperature of 250°C, and an outlet temperature of 110°C, and the obtained dry powder was subjected to preliminary calcination under conditions of 440°C and 4 hours. To the thus-obtained preliminary calcined powder (atomic ratio calculated based on the charged raw materials: Mo:Bi:Fe:Co:Ni:K = 12:1.0:1.7:6.0:2.8:0.07), a crystalline cellulose was added in an amount of 5 mass% with respect to the preliminary calcined powder, and the mixture was subjected to thorough mixing. Then, the resultant mixture was carried and molded in a spherical shape at an active mass ratio of 50 mass% on an inert carrier by using a 33 mass% glycerin solution as a binder by tumbling granulation. The thus-obtained spherical molded product having a particle diameter of 5.3 mm was subjected to main calcination under conditions of 520°C, 4 hours, and an air atmosphere to obtain a catalyst 1. A cumulative specific surface area and a pore volume distribution of the obtained catalyst were measured by mercury porosimetry, and a maximum value (A) of -dV/d (log D) in a pore diameter range of 0.45 $\mu$m to 10.0 $\mu$m and a maximum value (B) of -dV/d (log D) in a pore diameter range of 0.040 $\mu$m to 0.45 $\mu$m were respectively calculated. Specifically, the catalyst 1 was not subjected to pretreatment such as vacuum deaeration, a sample (weight of about 5 g) was put into a large cell (10 mm$\varphi$ $\times$ 6 cm) having a cell volume of 2 cc by using a fully automatic pore distribution analyzer (mercury porosimetry pore diameter analyzer Pore Master 60-GT manufactured by Anton Paar), mercury was filled into the cell, and a mercury pressure and a volume of the filled mercury were measured. Measurement was performed under conditions of a measurement temperature of 20°C and a measurement pore diameter range of 0.0036 $\mu$m to 400 $\mu$m by setting a mercury surface tension to 480 dyn/cm and a mercury contact angle to 140°, measurement results were analyzed using a pressure applied at the time of the measurement and the Washburn's equation regarding that all of the pores were cylindrical, to obtain the pore distribution of each pore diameter of the catalyst. Table 1 shows results of (B/A). Further, Table 2 shows results of a maximum value (C) of dV/dD in a range of a pore diameter of 0.30 $\mu$m to 10.0 $\mu$m, a maximum value (D) of dV/dD in a range of a pore diameter of 0.040 $\mu$m to 0.30 $\mu$m, (D/C), a maximum value (E) of dV in a range of a pore diameter of 0.35 $\mu$m to 100.0 $\mu$m, a maximum value (F) of dV in a range of a pore diameter of 0.040 $\mu$m to 0.35 $\mu$m, and (F/E).

(Measurement of Abrasion Resistance)

The catalyst 1 (50.0 g) was prepared and charged in a cylindrical rotating machine having a radius of 14 cm and including one baffle plate and the rotation was performed at 23 rpm for 10 minutes. Thereafter, the dropped powder was removed by a sieve having intervals of 1.7 mm, a remaining amount (g), which excludes the powder, was measured, and an abrasion resistance was 0.37% as being determined from the following formula.

$$\text{Abrasion resistance (\%)} = (50.0\ (g) - \text{remaining amount (g)})/50.0\ (g) \times 100$$

[Example 2]

In 380 parts by mass of pure water heated to 60°C, 100 parts by mass of ammonium heptamolybdate was completely dissolved (mother liquid 1). Next, 0.32 parts by mass of potassium nitrate was dissolved in 2.9 parts by mass of pure water, and the solution was added to the mother liquid 1. Next, 10.6 parts by mass of nitric acid (60 mass%) was added to the mother liquid 1 to adjust the pH to 4. Next, 36 parts by mass of ferric nitrate, 92 parts by mass of cobalt nitrate, and 27 parts by mass of nickel nitrate were dissolved in 83 parts by mass of pure water heated to 60°C, and the solution was added to the mother liquid 1. Subsequently, 18 parts by mass of bismuth nitrate was dissolved in a nitric acid aqueous solution prepared by adding 4.7 parts by mass of nitric acid (60 mass%) to 19 parts by mass of pure water heated to 60°C, and the solution was added to the mother liquid 1. The mother liquid 1 was dried by spray drying using a rotary atomizer under conditions of 13,500 rpm, an inlet temperature of 240°C, and an outlet temperature of 110°C, and the obtained dry powder was subjected to preliminary calcination under conditions of 440°C and 4 hours. To the thus-obtained preliminary calcined powder (atomic ratio calculated based on the charged raw materials: Mo:Bi:Fe:Co:Ni:K = 12:0.8:1.9:6.7:2.0:0.07), a crystalline cellulose was added in an amount of 5 mass% with respect to the preliminary calcined powder, and the mixture was subjected to thorough mixing. Then, the resultant mixture was carried and molded in a spherical shape at an active mass ratio of 50 mass% on an inert carrier by using a 33 mass% glycerin solution as a binder by tumbling granulation. The thus-obtained spherical molded product having a particle diameter of 5.3 mm was subjected to main calcination under conditions of 520°C, 4 hours, and an air atmosphere to obtain a catalyst 2. A cumulative specific surface area and a pore volume distribution of the catalyst obtained in the same manner as in Example 1 were measured by mercury porosimetry, and Table 1 shows results of (B/A). An abrasion resistance was 1.14% as determined by the same method as in Example 1. Further, Table 2 shows results of a maximum value (C) of dV/dD in a range of a pore diameter of 0.30 $\mu$m to 10.0 $\mu$m, a maximum value (D) of dV/dD in a range of a pore diameter of 0.040 $\mu$m to 0.30 $\mu$m, (D/C), a maximum value (E) of dV in a range of a pore diameter of 0.35 $\mu$m to 100.0 $\mu$m, a maximum value (F) of dV in a range of a pore diameter of 0.040 $\mu$m to 0.35 $\mu$m, and (F/E).

[Example 3]

In 380 parts by mass of pure water heated to 60°C, 100 parts by mass of ammonium heptamolybdate was completely dissolved (mother liquid 1). Next, 0.32 parts by mass of potassium nitrate was dissolved in 2.9 parts by mass of pure water, and the solution was added to the mother liquid 1. Next, 10.6 parts by mass of nitric acid (60 mass%) was added to the mother liquid 1 to adjust the pH to 4. Next, 31 parts by mass of ferric nitrate, 84 parts by mass of cobalt nitrate, and 40 parts by mass of nickel nitrate were dissolved in 82 parts by mass of pure water heated to 60°C, and the solution was added to the mother liquid 1. Subsequently, 18 parts by mass of bismuth nitrate was dissolved in a nitric acid aqueous solution prepared by adding 4.7 parts by mass of nitric acid (60 mass%) to 19 parts by mass of pure water heated to 60°C, and the solution was added to the mother liquid 1. The mother liquid 1 was dried by spray drying using a rotary atomizer under conditions of 14,000 rpm, an inlet temperature of 250°C, and an outlet temperature of 120°C, and the obtained dry powder was subjected to preliminary calcination under conditions of 440°C and 4 hours. To the thus-obtained preliminary calcined powder (atomic ratio calculated based on the charged raw materials: Mo:Bi:Fe:Co:Ni:K = 12:0.8:1.6:6.1:2.9:0.07), a crystalline cellulose was added in an amount of 5 mass% with respect to the preliminary calcined powder, and the mixture was subjected to thorough mixing. Then, the resultant mixture was carried and molded in a spherical shape at an active mass ratio of 50 mass% on an inert carrier by using a 33 mass% glycerin solution as a binder by tumbling granulation. The thus-obtained spherical molded product having a particle diameter of 5.3 mm was subjected to main calcination under conditions of 520°C and 4 hours to obtain a catalyst 3. A cumulative specific surface area and a pore volume distribution of the catalyst obtained in the same manner as in Example 1 were measured by mercury porosimetry, and Table 1 shows results of (B/A). An abrasion resistance was 0.77% as determined by the same method as in Example 1. Further, Table 2 shows results of a maximum value (C) of dV/dD in a range of a pore diameter of 0.30 μm to 10.0 μm, a maximum value (D) of dV/dD in a range of a pore diameter of 0.040 μm to 0.30 μm, (D/C), a maximum value (E) of dV in a range of a pore diameter of 0.35 μm to 100.0 μm, a maximum value (F) of dV in a range of a pore diameter of 0.040 μm to 0.35 μm, and (F/E).

[Reference Example 1]

In 380 parts by mass of pure water heated to 60°C, 100 parts by mass of ammonium heptamolybdate was completely dissolved (mother liquid 1). Next, 0.45 parts by mass of potassium nitrate was dissolved in 45 parts by mass of pure water, and the solution was added to the mother liquid 1. Next, 38 parts by mass of ferric nitrate, 71 parts by mass of cobalt nitrate, and 38 parts by mass of nickel nitrate were dissolved in 76 parts by mass of pure water heated to 60°C, and the solution was added to the mother liquid 1. Subsequently, 36 parts by mass of bismuth nitrate was dissolved in a nitric acid aqueous solution prepared by adding 9.7 parts by mass of nitric acid (60 mass%) to 41 parts by mass of pure water heated to 60°C, and the solution was added to the mother liquid 1. The mother liquid 1 was dried by spray drying using a rotary atomizer under conditions of 14,000 rpm, an inlet temperature of 240°C, and an outlet temperature of 115°C, and the obtained dry powder was subjected to preliminary calcination under conditions of 440°C and 4 hours. To the thus-obtained preliminary calcined powder (atomic ratio calculated based on the charged raw materials: Mo:Bi:Fe:Co:Ni:K = 12:1.6:2.0:5.2:2.8:0.10), a crystalline cellulose was added in an amount of 5 mass% with respect to the preliminary calcined powder, the mixture was subjected to thorough mixing. Then, the resultant mixture was carried and molded in a spherical shape at an active mass ratio of 50 mass% on an inert carrier by using a 33 mass% glycerin solution as a binder by tumbling granulation. The thus-obtained spherical molded product having a particle diameter of 5.3 mm was subjected to main calcination under conditions of 530°C, 4 hours, and an air atmosphere to obtain a catalyst 4.

[Comparative Example 1]

In 380 parts by mass of pure water heated to 60°C, 100 parts by mass of ammonium heptamolybdate was completely dissolved (mother liquid 1). Next, 0.17 parts by mass of potassium nitrate was dissolved in 1.9 parts by mass of pure water, and the solution was added to the mother liquid 1. Next, 41 parts by mass of ferric nitrate, 89 parts by mass of cobalt nitrate, and 33 parts by mass of nickel nitrate were dissolved in 85 parts by mass of pure water heated to 60°C, and the solution was added to the mother liquid 1. Subsequently, 16 parts by mass of bismuth nitrate was dissolved in a nitric acid aqueous solution prepared by adding 4.1 parts by mass of nitric acid (60 mass%) to 17 parts by mass of pure water heated to 60°C, and the solution was added to the mother liquid 1. This mother liquid 1 was dried by spray drying, and the obtained dry powder was subjected to preliminary calcination under conditions of 440°C and 4 hours. To the thus-obtained preliminary calcined powder (atomic ratio calculated based on the charged raw materials: Mo:Bi:Fe:Co:Ni:K = 12:0.7:2.2:6.5:2.4:0.04), a crystalline cellulose was added in an amount of 5 mass% with respect to the preliminary calcined powder, and the mixture was subjected to thorough mixing. Then, the resultant mixture was carried and molded in a spherical shape at an active mass ratio of 50 mass% on an inert carrier by using a 33 mass% glycerin solution as a binder by tumbling granulation. The thus-obtained spherical molded product having a particle diameter of 5.3 mm was subjected to main calcination under conditions of 550°C and 4 hours to obtain a catalyst 5. A cumulative specific surface area and a pore volume distribution of the catalyst obtained in the same manner as in Example 1 were measured by mercury porosimetry, and Table 1 shows results of (B/A). Further, Table 2 shows results of a maximum value (C) of dV/dD in a range of a pore diameter of 0.30 μm to 10.0 μm, a maximum value (D) of dV/dD in a range of a pore diameter of 0.040 μm to 0.30 μm, (D/C), a maximum value (E) of dV in a range of a pore diameter of 0.35 μm to 100.0 μm, a maximum value (F) of dV in a range of a pore diameter of 0.040 μm to 0.35 μm, and (F/E).

The oxidation reaction of propylene was carried out by the following method using the catalyst 1 to the catalyst 3 and the catalyst 5, and the raw material conversion rate and the useful yield were determined. A gas inlet side of a stainless steel reaction tube having an inner diameter of 28 mm was filled with 33.8 mL of the catalyst 4 and a gas outlet side was filled with 47.3 mL of any one of the catalyst 1 to the catalyst 3 and the catalyst 5. A mixed gas containing propylene, oxygen, water vapor, and nitrogen in a gas volume ratio of 1.00:1.65:1.27:9.53 was introduced into the reaction tube at a propylene space velocity of 100 hr$^{-1}$ with respect to all the catalysts, to carry out the oxidation reaction of propylene. An aging reaction was carried out at a salt bath temperature of 315°C for 20 hours or longer from the start of the reaction, and then, the gas at the outlet of the reaction tube was analyzed at a salt bath temperature of 320°C to obtain the raw material conversion rate and the useful yield shown in Table 1.

[Table 1]

| Example | Catalyst | A | B | B/A | Cumulative specific surface area (m²/g) | Propylene conversion rate (%) | Useful selectivity (%) | Useful yield (%) |
|---|---|---|---|---|---|---|---|---|
| Example 1 | Catalyst 1 | 0.13 | 0.19 | 1.46 | 1.2 | 97.1 | 94.6 | 91.8 |
| Example 2 | Catalyst 2 | 0.81 | 0.31 | 0.38 | 3.0 | 968 | 94.9 | 91.9 |
| Example 3 | Catalyst 3 | 0.37 | 0.25 | 0.68 | 1.7 | 97.3 | 94.7 | 92.2 |
| Comparative example 1 | Catalyst 5 | 1.97 | 0.20 | 0.10 | 2.6 | 97.6 | 93.8 | 91.5 |

[Table 2]

| Example | Catalyst | C | D | D/C | E | F | F/E |
|---|---|---|---|---|---|---|---|
| Example 1 | Catalyst 1 | 0.07 | 0.29 | 4.14 | 0.0006 | 0.0014 | 2.33 |
| Example 2 | Catalyst 2 | 0.57 | 0.66 | 1.16 | 0.0029 | 0.0016 | 0.55 |
| Example 3 | Catalyst 3 | 0.17 | 0.40 | 2.35 | 0.0016 | 0.0016 | 1.00 |
| Comparative example 1 | Catalyst 5 | 1.33 | 0.48 | 0.36 | 0.0045 | 0.0010 | 0.22 |

[Example 4]

In 380 parts by mass of pure water heated to 60°C, 100 parts by mass of ammonium heptamolybdate was completely dissolved (mother liquid 1). Next, 0.37 parts by mass of cesium nitrate was dissolved in 3.3 parts by mass of pure water, and the solution was added to the mother liquid 1. Next, 10.6 parts by mass of nitric acid (60 mass%) was added to the mother liquid 1 to adjust the pH to 4. Next, 38 parts by mass of ferric nitrate, 85 parts by mass of cobalt nitrate, and 27 parts by mass of nickel nitrate were dissolved in 80 parts by mass of pure water heated to 60°C, and the solution was added to the mother liquid 1. Subsequently, 18 parts by mass of bismuth nitrate was dissolved in a nitric acid aqueous solution prepared by adding 4.7 parts by mass of nitric acid (60 mass%) to 19 parts by mass of pure water heated to 60°C, and the solution was added to the mother liquid 1. The mother liquid 1 was dried by spray drying using a rotary atomizer under conditions of 14,000 rpm, an inlet temperature of 240°C, and an outlet temperature of 110°C, and the obtained dry powder was subjected to preliminary calcination under conditions of 440°C and 4 hours. To the thus-obtained preliminary calcined powder (atomic ratio calculated based on the charged raw materials: Mo:Bi:Fe:Co:Ni:Cs = 12:0.8:2.0:6.2:2.0:0.04), a crystalline cellulose was added in an amount of 5 mass% with respect to the preliminary calcined powder, and the mixture was subjected to thorough mixing. Then, the resultant mixture was carried and molded in a spherical shape at an active mass ratio of 50 mass% on an inert carrier by using a 33 mass% glycerin solution as a binder by tumbling granulation. The thus-obtained spherically molded product having a particle diameter of 5.3 mm was subjected to main calcination under conditions of 500°C, 4 hours, and an air atmosphere to obtain a catalyst 6. A cumulative specific surface area and a pore volume distribution of the catalyst obtained in the same manner as in Example 1 were measured by mercury porosimetry, and Table 3 shows results of (B/A). An abrasion resistance was 0.98% as determined by the same method as in Example 1. Further, Table 4 shows results of a maximum value (C) of dV/dD in a range of a pore diameter of 0.30 μm to 10.0 μm, a maximum value (D) of dV/dD in a range of a pore diameter of 0.040 μm to 0.30 μm, (D/C), a maximum value (E) of dV in a range of a pore diameter of 0.35 μm to 100.0 μm, a maximum value (F) of dV in a range of a pore diameter of 0.040 μm to 0.35 μm, and (F/E).

[Example 5]

In 380 parts by mass of pure water heated to 60°C, 100 parts by mass of ammonium heptamolybdate was completely dissolved (mother liquid 1). Next, 0.46 parts by mass of cesium nitrate was dissolved in 4.1 parts by mass of pure water, and the solution was added to the mother liquid 1. Subsequently, 0.27 parts by mass of phosphoric acid (85 mass%) was added to the mother liquid 1. Next, 31 parts by mass of ferric nitrate, 85 parts by mass of cobalt nitrate, and 41 parts by mass of nickel nitrate were dissolved in 83 parts by mass of pure water heated to 60°C, and the solution was added to the mother liquid 1. Subsequently, 20 parts by mass of bismuth nitrate was dissolved in a nitric acid aqueous solution prepared by adding 5.0 parts by mass of nitric acid (60 mass%) to 21 parts by mass of pure water heated to 60°C, and the solution was added to the mother liquid 1. The mother liquid 1 was dried by spray drying using a rotary atomizer under conditions of 13,500 rpm, an inlet temperature of 240°C, and an outlet temperature of 110°C, and the obtained dry powder was subjected to preliminary calcination under conditions of 440°C and 4 hours. To the thus-obtained preliminary calcined powder (atomic ratio calculated based on the charged raw materials: $Mo:Bi:Fe:Co:Ni:Cs = 12:0.9:1.6:6.2:3.0:0.05$), a crystalline cellulose was added in an amount of 5 mass% with respect to the preliminary calcined powder, and the mixture was subjected to thorough mixing. Then, the resultant mixture was carried and molded in a spherical shape at an active mass ratio of 50 mass% on an inert carrier by using a 33 mass% glycerin solution as a binder by tumbling granulation. The thus-obtained spherically molded product having a particle diameter of 5.3 mm was subjected to main calcination under conditions of 505°C, 4 hours, and an air atmosphere to obtain a catalyst 7. A cumulative specific surface area and a pore volume distribution of the catalyst obtained in the same manner as in Example 1 were measured by mercury porosimetry, and Table 3 shows results of (B/A). An abrasion resistance was 1.02% as determined by the same method as in Example 1. Further, Table 4 shows results of a maximum value (C) of dV/dD in a range of a pore diameter of 0.30 µm to 10.0 µm, a maximum value (D) of dV/dD in a range of a pore diameter of 0.040 µm to 0.30 µm, (D/C), a maximum value (E) of dV in a range of a pore diameter of 0.35 µm to 100.0 µm, a maximum value (F) of dV in a range of a pore diameter of 0.040 µm to 0.35 µm, and (F/E).

[Example 6]

In 380 parts by mass of pure water heated to 60°C, 100 parts by mass of ammonium heptamolybdate was completely dissolved (mother liquid 1). Next, 0.46 parts by mass of cesium nitrate was dissolved in 4.1 parts by mass of pure water, and the solution was added to the mother liquid 1. Next, 10.6 parts by mass of nitric acid (60 mass%) was added to the mother liquid 1 to adjust the pH to 4. Subsequently, 0.33 parts by mass of phosphoric acid (85 mass%) was added to the mother liquid 1. Next, 30 parts by mass of ferric nitrate, 83 parts by mass of cobalt nitrate, and 43 parts by mass of nickel nitrate were dissolved in 83 parts by mass of pure water heated to 60°C, and the solution was added to the mother liquid 1. Subsequently, 22 parts by mass of bismuth nitrate was dissolved in a nitric acid aqueous solution prepared by adding 5.5 parts by mass of nitric acid (60 mass%) to 23 parts by mass of pure water heated to 60°C, and the solution was added to the mother liquid 1. The mother liquid 1 was dried by spray drying using a rotary atomizer under conditions of 15,000 rpm, an inlet temperature of 250°C, and an outlet temperature of 110°C, and the obtained dry powder was subjected to preliminary calcination under conditions of 440°C and 4 hours. To the thus-obtained preliminary calcined powder (atomic ratio calculated based on the charged raw materials: $Mo:Bi:Fe:Co:Ni:Cs = 12:0.9:1.6:6.1:3.2:0.05$), a crystalline cellulose was added in an amount of 5 mass% with respect to the preliminary calcined powder, and the mixture was subjected to thorough mixing. Then, the resultant mixture was carried and molded in a spherical shape at an active mass ratio of 50 mass% on an inert carrier by using a 33 mass% glycerin solution as a binder by tumbling granulation. The thus-obtained spherically molded product having a particle diameter of 5.3 mm was subjected to main calcination under conditions of 515°C, 4 hours, and an air atmosphere to obtain a catalyst 8. A cumulative specific surface area and a pore volume distribution of the catalyst obtained in the same manner as in Example 1 were measured by mercury porosimetry, and Table 3 shows results of (B/A). An abrasion resistance was 0.77% as determined by the same method as in Example 1. Further, Table 4 shows results of a maximum value (C) of dV/dD in a range of a pore diameter of 0.30 µm to 10.0 µm, a maximum value (D) of dV/dD in a range of a pore diameter of 0.040 µm to 0.30 µm, (D/C), a maximum value (E) of dV in a range of a pore diameter of 0.35 µm to 100.0 µm, a maximum value (F) of dV in a range of a pore diameter of 0.040 µm to 0.35 µm, and (F/E).

[Example 7]

In 380 parts by mass of pure water heated to 60°C, 100 parts by mass of ammonium heptamolybdate was completely dissolved (mother liquid 1). Next, 0.46 parts by mass of cesium nitrate was dissolved in 4.1 parts by mass of pure water, and the solution was added to the mother liquid 1. Subsequently, 0.22 parts by mass of phosphoric acid (85 mass%) was added to the mother liquid 1. Next, 34 parts by mass of ferric nitrate, 91 parts by mass of cobalt nitrate, and 40 parts by mass of nickel nitrate were dissolved in 87 parts by mass of pure water heated to 60°C, and the solution was added to the mother liquid 1. Subsequently, 20 parts by mass of bismuth nitrate was dissolved in a nitric acid aqueous solution prepared by adding 5.0 parts by mass of nitric acid (60 mass%) to 21 parts by mass of pure water heated to 60°C, and the solution was added to the mother liquid 1. The mother liquid 1 was dried by spray drying using a rotary atomizer under conditions of 14,500 rpm, an inlet temperature of 255°C, and an outlet temperature of 115°C, and the obtained dry powder was subjected to preliminary calcination under conditions of 440°C and 4 hours. To the thus-obtained preliminary calcined powder (atomic ratio calculated based on the charged raw materials: Mo:Bi:Fe:Co:Ni:Cs = 12:0.9:1.8:6.6:2.9:0.05), a crystalline cellulose was added in an amount of 5 mass% with respect to the preliminary calcined powder, and the mixture was subjected to thorough mixing. Then, the resultant mixture was carried and molded in a spherical shape at an active mass ratio of 50 mass% on an inert carrier by using a 33 mass% glycerin solution as a binder by tumbling granulation. The thus-obtained spherically molded product having a particle diameter of 5.3 mm was subjected to main calcination under conditions of 530°C, 4 hours, and an air atmosphere to obtain a catalyst 9. A cumulative specific surface area and a pore volume distribution of the catalyst obtained in the same manner as in Example 1 were measured by mercury porosimetry, and Table 3 shows results of (B/A). An abrasion resistance was 0.91% as determined by the same method as in Example 1. Further, Table 4 shows results of a maximum value (C) of dV/dD in a range of a pore diameter of 0.30 $\mu$m to 10.0 $\mu$m, a maximum value (D) of dV/dD in a range of a pore diameter of 0.040 $\mu$m to 0.30 $\mu$m, (D/C), a maximum value (E) of dV in a range of a pore diameter of 0.35 $\mu$m to 100.0 $\mu$m, a maximum value (F) of dV in a range of a pore diameter of 0.040 $\mu$m to 0.35 $\mu$m, and (F/E).

[Example 8]

In 380 parts by mass of pure water heated to 60°C, 100 parts by mass of ammonium heptamolybdate was completely dissolved (mother liquid 1). Next, 0.37 parts by mass of cesium nitrate was dissolved in 3.3 parts by mass of pure water, and the solution was added to the mother liquid 1. Next, 38 parts by mass of ferric nitrate, 85 parts by mass of cobalt nitrate, and 30 parts by mass of nickel nitrate were dissolved in 81 parts by mass of pure water heated to 60°C, and the solution was added to the mother liquid 1. Subsequently, 18 parts by mass of bismuth nitrate was dissolved in a nitric acid aqueous solution prepared by adding 4.7 parts by mass of nitric acid (60 mass%) to 19 parts by mass of pure water heated to 60°C, and the solution was added to the mother liquid 1. The mother liquid 1 was dried by spray drying using a rotary atomizer under conditions of 14,000 rpm, an inlet temperature of 240°C, and an outlet temperature of 110°C, and the obtained dry powder was subjected to preliminary calcination under conditions of 440°C and 4 hours. To the thus-obtained preliminary calcined powder (atomic ratio calculated based on the charged raw materials: Mo:Bi:Fe:Co:Ni:Cs = 12:0.8:2.0:6.2:2.2:0.04), a crystalline cellulose was added in an amount of 5 mass% with respect to the preliminary calcined powder, and the mixture was subjected to thorough mixing. Then, the resultant mixture was carried and molded in a spherical shape at an active mass ratio of 50 mass% on an inert carrier by using a 33 mass% glycerin solution as a binder by tumbling granulation. The thus-obtained spherically molded product having a particle diameter of 5.3 mm was subjected to main calcination under conditions of 500°C, 4 hours, and an air atmosphere to obtain a catalyst 10. A cumulative specific surface area and a pore volume distribution of the catalyst obtained in the same manner as in Example 1 were measured by mercury porosimetry, and Table 3 shows results of (B/A). An abrasion resistance was 0.68% as determined by the same method as in Example 1. Further, Table 4 shows results of a maximum value (C) of dV/dD in a range of a pore diameter of 0.30 $\mu$m to 10.0 $\mu$m, a maximum value (D) of dV/dD in a range of a pore diameter of 0.040 $\mu$m to 0.30 $\mu$m, (D/C), a maximum value (E) of dV in a range of a pore diameter of 0.35 $\mu$m to 100.0 $\mu$m, a maximum value (F) of dV in a range of a pore diameter of 0.040 $\mu$m to 0.35 $\mu$m, and (F/E).

[Comparative Example 2]

In 380 parts by mass of pure water heated to 60°C, 100 parts by mass of ammonium heptamolybdate was completely dissolved (mother liquid 1). Next, 0.37 parts by mass of cesium nitrate was dissolved in 3.3 parts by mass of pure water, and the solution was added to the mother liquid 1. Next, 37 parts by mass of ferric nitrate, 85 parts by mass of cobalt nitrate, and 36 parts by mass of nickel nitrate were dissolved in 84 parts by mass of pure water heated to 60°C, and the solution was added to the mother liquid 1. Subsequently, 21 parts by mass of bismuth nitrate was dissolved in a nitric acid aqueous solution prepared by adding 5.4 parts by mass of nitric acid (60 mass%) to 23 parts by mass of pure water heated to 60°C, and the solution was added to the mother liquid 1. This mother liquid 1 was dried by spray drying, and the obtained dry powder was subjected to preliminary main calcination under conditions of 440°C and 4 hours. To the thus-obtained preliminary calcined powder (atomic ratio calculated based on the charged raw materials: Mo:Bi:Fe:Co:Ni:Cs = 12:0.9:2.0:6.2:2.6:0.04), a crystalline cellulose was added in an amount of 5 mass% with respect to the preliminary calcined powder, and the mixture was subjected to thorough mixing. Then, the resultant mixture was carried and molded in a spherical shape at an active mass ratio of 50 mass% on an inert carrier by using a 33 mass% glycerin solution as a binder by tumbling granulation. The thus-obtained spherically molded product having a particle diameter of 5.3 mm was subjected to main calcination under conditions of 540°C and 4 hours to obtain a catalyst 11. A cumulative specific surface area and a pore volume distribution of the catalyst obtained in the same manner as in Example 1 were measured by mercury porosimetry, and Table 3 shows results of (B/A). Further, Table 4 shows results of a maximum value (C) of dV/dD in a range of a pore diameter of 0.30 $\mu$m to 10.0 $\mu$m, a maximum value (D) of dV/dD in a range of a pore diameter of 0.040 $\mu$m to 0.30 $\mu$m, (D/C), a maximum value (E) of dV in a range of a pore diameter of 0.35 $\mu$m to 100.0 $\mu$m, a maximum value (F) of dV in a range of a pore diameter of 0.040 $\mu$m to 0.35 $\mu$m,

and (F/E).

The oxidation reaction of propylene was carried out by the following method using the catalyst 6 to the catalyst 11, and the raw material conversion rate and the useful yield were determined. A gas inlet side of a stainless steel reaction tube having an inner diameter of 22 mm was filled with 50.7 mL of any one of the catalyst 6 to the catalyst 11. A mixed gas containing propylene, oxygen, water vapor, and nitrogen in a gas volume ratio of 1.00:1.70:1.00:8.80 was introduced into the reaction tube at a propylene space velocity of 100 hr' with respect to all the catalysts, to carry out the oxidation reaction of propylene. An aging reaction was carried out at a salt bath temperature of 315°C for 20 hours or longer from the start of the reaction, and then, the gas at the outlet of the reaction tube was analyzed at a salt bath temperature of 320°C to obtain the raw material conversion rate and the useful yield shown in Table 3.

[Table 3]

| Example | Catalyst | A | B | B/A | Cumulative specific surface area ($m^2/g$) | Propylene conversion rate (%) | Useful selectivity (%) | Useful yield (%) |
|---|---|---|---|---|---|---|---|---|
| Example 4 | Catalyst 6 | 0.31 | 0.37 | 1.16 | 2.7 | 78.4 | 97.3 | 76.3 |
| Example 5 | Catalyst 7 | 0.63 | 1.13 | 1.79 | 2.8 | 79.3 | 97.0 | 76.9 |
| Example 6 | Catalyst 8 | 0.48 | 0.34 | 0.71 | 2.2 | 80.0 | 96.9 | 77.6 |
| Example 7 | Catalyst 9 | 1.22 | 1.50 | 1.24 | 2.7 | 78.5 | 96.8 | 76.0 |
| Example 8 | Catalyst 10 | 0.41 | 0.37 | 0.91 | 2.7 | 79.0 | 96.9 | 76.5 |
| Comparative example 2 | Catalyst 11 | 0.13 | 1.66 | 13.21 | 3.0 | 78.4 | 96.8 | 75.8 |

[Table 4]

| Example | Catalyst | C | D | D/C | E | F | F/E |
|---|---|---|---|---|---|---|---|
| Example 4 | Catalyst 6 | 0.19 | 0.82 | 4.25 | 0.0019 | 0.0039 | 2.05 |
| Example 5 | Catalyst 7 | 1.20 | 0.42 | 0.35 | 0.0118 | 0.0047 | 0.40 |
| Example 6 | Catalyst 8 | 0.48 | 0.53 | 1.11 | 0.0027 | 0.0020 | 0.74 |
| Example 7 | Catalyst 9 | 1.50 | 0.50 | 0.33 | 0.0069 | 0.0019 | 0.28 |
| Example 8 | Catalyst 10 | 0.26 | 0.82 | 3.09 | 0.0013 | 0.0019 | 1.46 |
| Comparative example 2 | Catalyst 11 | 2.17 | 0.74 | 0.34 | 0.0036 | 0.0057 | 1.58 |

It has been found from Tables 1 and 3 that the catalysts 1 to 3 and the catalysts 6 to 10 in which (B/A) is within the range of the present invention exhibited significantly high useful yield. Further, it has been found that these catalysts had an abrasion resistance of 3% or less and thus had strength to withstand practical use.

The present application is based on a Japanese Patent Application No. 2021-193719 filed on Nov. 30, 2021, contents of which are incorporated herein by reference.

INDUSTRIAL APPLICABILITY

By using the catalyst according to the present invention, when an unsaturated aldehyde compound, an unsaturated carboxylic acid compound, or a conjugated diene compound is produced in an oxidative manner, the compound may be obtained in a region having a high catalytic activity with a high yield.

**Claims**

1. A catalyst having at least one peak in a range of a pore diameter of 0.45 μm to 10.0 μm and at least one peak in a range of a pore diameter of 0.040 μm to 0.45 μm in a pore distribution (-dV/d (log D) vs D) measured by mercury porosimetry, wherein
when a maximum value of -dV/d (log D) in the range of the pore diameter of 0.45 μm to 10.0 μm is represented by A and a maximum value of -dV/d (log D) in the range of the pore diameter of 0.040 μm to 0.45 μm is represented by B, (B/A) is 0.20 to 13.0, where V represents a volume (unit: mL/g) of filled mercury per sample weight, D represents the pore diameter (unit: μm), log D represents a natural logarithm of D, and -dV/d (log D) represents a reciprocal of a value obtained by differentiating a cumulative pore volume in a specific pore diameter range by a logarithm of the pore diameter.

2. The catalyst according to claim 1, wherein a cumulative specific surface area is less than 5 $m^2/g$.

3. The catalyst according to claim 1 or 2, comprising molybdenum, bismuth, and iron.

4. The catalyst according to claim 3, comprising a catalyst composition represented by the following formula (1):

$$Mo_{a1}Bi_{b1}Ni_{c1}Co_{d1}Fe_{e1}X_{f1}Y_{g1}Z_{h1}O_{i1} \qquad (1),$$

where, Mo, Bi, Ni, Co, and Fe respectively represent molybdenum, bismuth, nickel, cobalt, and iron; X is at least one element selected from tungsten, antimony, tin, zinc, chromium, manganese, magnesium, silicon, aluminum, cerium, and titanium; Y is at least one element selected from sodium, potassium, cesium, rubidium, and thallium; Z belongs to Groups 1 to 16 in the periodic table, and means at least one element selected from elements other than the above Mo, Bi, Ni, Co, Fe, X, and Y; a1, b1, c1, d1, e1, f1, g1, h1, and i1 respectively represent the number of atoms of molybdenum, bismuth, nickel, cobalt, iron, X, Y, Z, and oxygen; and when a1 = 12, $0 < b1 \leq 7$, $0 \leq c1 \leq 10$, $0 < d1 \leq 10$, $0 < c1+d1 \leq 20$, $0 < e1 \leq 5$, $0 \leq f1 \leq 2$, $0 \leq g1 \leq 3$, $0 \leq h1 \leq 5$, and i1 is a value determined by an oxidation state of each element.

5. The catalyst according to any one of claims 1 to 4, wherein the catalyst is carried on an inert carrier.

6. The catalyst according to claim 5, wherein the inert carrier is silica and/or alumina.

7. The catalyst according to any one of claims 1 to 6, wherein the catalyst is used for producing an unsaturated aldehyde compound, an unsaturated carboxylic acid compound, or a conjugated diene compound.

8. A method for producing an unsaturated aldehyde compound, an unsaturated carboxylic acid compound, or a conjugated diene compound, comprising using the catalyst according to any one of claims 1 to 7.

9. The method according to claim 8, wherein the unsaturated aldehyde compound is acrolein, the unsaturated carboxylic acid compound is acrylic acid, and the conjugated diene compound is butadiene.

## FIG. 1

# FIG. 2

## INTERNATIONAL SEARCH REPORT

| | International application No. |
|---|---|
| | **PCT/JP2022/043923** |

**A. CLASSIFICATION OF SUBJECT MATTER**

***B01J 23/887***(2006.01)i; ***B01J 35/10***(2006.01)i; ***C07B 61/00***(2006.01)i; ***C07C 45/35***(2006.01)i; ***C07C 47/22***(2006.01)i
FI:    B01J23/887 Z; B01J35/10 301G; C07C45/35; C07C47/22 A; C07B61/00 300

According to International Patent Classification (IPC) or to both national classification and IPC

**B. FIELDS SEARCHED**

Minimum documentation searched (classification system followed by classification symbols)

B01J21/00-38/74; C07B61/00; C07C1/00-409/44

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2007-505740 A (BASF AKTIENGESELLSCHAFT) 15 March 2007 (2007-03-15) claims 20, 28, examples, table 1, fig. 7-9, 11, 16 | 1, 3, 4, 7-9 |
| A | | 2, 5, 6 |
| X | JP 2008-86877 A (NIPPON SHOKUBAI CO LTD) 17 April 2008 (2008-04-17) claim 1, paragraphs [0017]-[0018], example 1, fig. 1A, B | 1, 2, 5, 6 |
| A | | 3, 4, 7-9 |
| X | JP 2010-29844 A (JGC CORP) 12 February 2010 (2010-02-12) claims 1, 3, examples 3, 6, fig. 2 | 1, 2, 5, 6 |
| A | | 3, 4, 7-9 |
| A | JP 2007-511565 A (BASF AKTIENGESELLSCHAFT) 10 May 2007 (2007-05-10) claim 1, examples, fig. 1 | 1-9 |

☐ Further documents are listed in the continuation of Box C.      ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **18 January 2023** | **07 February 2023** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

<table>
<tr><td colspan="2" align="center"><strong>INTERNATIONAL SEARCH REPORT</strong><br>Information on patent family members</td><td colspan="2">International application No.<br><strong>PCT/JP2022/043923</strong></td></tr>
</table>

| Patent document cited in search report | Publication date (day/month/year) | Patent family member(s) | Publication date (day/month/year) |
|---|---|---|---|
| JP 2007-505740 A | 15 March 2007 | US 2005/0065371 A1 claims 20, 28, examples, tables, fig. 7-9, 11, 16<br>EP 1663488 A1<br>CN 1856364 A<br>KR 10-2006-0099509 A | |
| JP 2008-86877 A | 17 April 2008 | US 2008/0091038 A1 claim 1, paragraphs [0035]-[0036], example 1, fig. 1A, B<br>EP 1927398 A1<br>KR 10-2008-0030509 A<br>CN 101396659 A | |
| JP 2010-29844 A | 12 February 2010 | US 2010/0207069 A1 claims 1, 3, examples 3, 6, fig. 2<br>WO 2010/001539 A1<br>EP 2298447 A1 | |
| JP 2007-511565 A | 10 May 2007 | US 2005/0107641 A1 claim 1, examples, fig. 1<br>WO 2005/049200 A1<br>EP 1689524 A1<br>CN 1882385 A<br>KR 10-2006-0132585 A | |

Form PCT/ISA/210 (patent family annex) (January 2015)

**REFERENCES CITED IN THE DESCRIPTION**

**Patent documents cited in the description**

- WO 2016136882 A
- JP 2017024009 A
- JP H06007924 B
- JP 6447327 B
- JP 5970542 B
- JP 2021193719 A